# EUROPEAN PATENT SPECIFICATION

(11) **EP 4 346 939 B1**
(45) Date of publication and mention of the grant of the patent: **02.12.2026**
(21) Application number: 22816692.2
(22) Date of filing: 27.05.2022
(51) Int. Cl.: A61M 1/14, A61M 1/34, A61M 1/16, C01C 1/02, C01D 13/00

(54) **METHODS AND SYSTEMS FOR CONTROLLING DIALYSATE SALT CONCENTRATION**
VERFAHREN UND SYSTEME ZUR STEUERUNG DER DIALYSATSALZKONZENTRATION
PROCÉDÉS ET SYSTÈMES DE RÉGULATION DE LA CONCENTRATION EN SEL D'UN DIALYSAT

(30) Priority: 31.05.2021 US 202163195164 P
(43) Date of publication of application: 10.04.2024
(73) Proprietor: Diality Inc., Irvine CA 92618 (US)
(72) Inventor: BORILLO, Brandon, Irvine, CA 92618 (US); POPPE, Clayton, Irvine, CA 92618 (US)
(74) Representative: Dolleymores
(86) International application number: PCT/US2022/031454
(87) International publication number: WO 2022/256269

(56) References cited:
- EP-B1- 3 397 303
- WO-A1-2012/148781
- US-A- 4 202 760
- US-A1- 2011 017 665
- US-A1- 2014 190 876
- US-A1- 2014 190 886
- US-A1- 2017 043 078
- US-A1- 2017 281 847
- US-A1- 2020 129 686

## Description

### CROSS-REFERENCE TO RELATED APPLICATIONS

The present application claims priority to, and the benefit of, U.S. Provisional Patent Application No. 63/195,164, filed May 31, 2021.

### BACKGROUND OF THE INVENTION

The present invention relates to an artificial kidney system for use in providing dialysis. More particularly, the present invention is directed to a hemodialysis system having a system for replenishing essential minerals in the dialysate.

Hemodialysis is a medical procedure that is used to achieve the extracorporeal removal of waste products including creatine, urea, and free water from a patient's blood involving the diffusion of solutes across a semipermeable membrane. Failure to properly remove these waste products can result in renal failure.

During hemodialysis, the patient's blood is removed by an arterial line, treated by a dialysis machine, and returned to the body by a venous line. The dialysis machine includes a dialyzer containing a large number of hollow fibers forming a semipermeable membrane through which the blood is transported. In addition, the dialysis machine utilizes a dialysate liquid, containing the proper amounts of electrolytes and other essential constituents (such as glucose), that is also pumped through the dialyzer.

Typically, dialysate is prepared by mixing water with appropriate proportions of an acid concentrate and a bicarbonate concentrate. Preferably, the acid and the bicarbonate concentrate are separated until the final mixing right before use in the dialyzer as the calcium and magnesium in the acid concentrate will precipitate out when in contact with the high bicarbonate level in the bicarbonate concentrate. The dialysate may also include appropriate levels of sodium, potassium, chloride, and glucose.

The dialysis process across the membrane is achieved by a combination of diffusion and convection. The diffusion entails the migration of molecules by random motion from regions of high concentration to regions of low concentration. Meanwhile, convection entails the movement of solute typically in response to a difference in hydrostatic pressure. The fibers forming the semipermeable membrane separate the blood plasma from the dialysate and provide a large surface area for diffusion to take place which allows waste, including urea, potassium and phosphate, to permeate into the dialysate while preventing the transfer of larger molecules such as blood cells, polypeptides, and certain proteins into the dialysate.

Typically, the dialysate flows in the opposite direction to blood flow in the extracorporeal circuit. The countercurrent flow maintains the concentration gradient across the semipermeable membrane so as to increase the efficiency of the dialysis. In some instances, hemodialysis may provide for fluid removal, also referred to as ultrafiltration. Ultrafiltration is commonly accomplished by lowering the hydrostatic pressure of the dialysate compartment of a dialyzer, thus allowing water containing dissolved solutes, including electrolytes and other permeable substances, to move across the membrane from the blood plasma to the dialysate. In rarer circumstances, fluid in the dialysate flow path portion of the dialyzer is higher than the blood flow portion, causing fluid to move from the dialysis flow path to the blood flow path. This is commonly referred to as reverse ultrafiltration. Since ultrafiltration and reverse ultrafiltration can increase the risks to a patient, ultrafiltration and reverse ultrafiltration are typically conducted while supervised by highly trained medical personnel.

Unfortunately, hemodialysis suffers from numerous drawbacks. An arteriovenous fistula is the most commonly recognized access point. To create a fistula, a doctor joins an artery and a vein together. Since this bypasses the patient's capillaries, blood flows rapidly. For each dialysis session, the fistula must be punctured with large needles to deliver blood into, and return blood from, the dialyzer. Typically, this procedure is done three times a week, for 3 - 4 hours at an out-patient facility. To a lesser extent, patients conduct hemodialysis at home. Some forms of home dialysis are done for two hours, six days a week. Other forms use two and a half to three hour treatments, four to 5 days a week. Currently offered home hemodialysis requires more frequent treatments than those in an out-patient setting.

Relevant prior art is for instance disclosed in documents US2020/129686 A1, WO2012/148781 A1, US2011/017665 A1 and EP3397303 B1.

Home hemodialysis suffers from still additional disadvantages. Current home dialysis systems are big, complicated, intimidating and difficult to operate. The equipment requires significant training. Home hemodialysis systems are currently too large to be portable, thereby preventing hemodialysis patients from traveling. Home hemodialysis systems are expensive and require a high initial monetary investment, particularly compared to in-center hemodialysis where patients are not required to pay for the machinery. Present home hemodialysis systems do not adequately provide for the reuse of supplies, making home hemodialysis economically less feasible to medical suppliers. As a result of the above-mentioned disadvantages, very few motivated patients undertake the drudgery of home hemodialysis.

Accordingly, there is a significant need for a hemodialysis system that is transportable, lightweight, easy to use, patient-friendly and thus capable of in-clinic or in-home use.

Moreover, it would be desirable to provide a hemodialysis system that possessed no single-point of failure in the pumps, motors, tubes, or electronics which would endanger a patient.

In addition, it would be desirable to provide a hemodialysis system that was capable of being used in a variety of modes, such as with a filter to cleanse dialysate or without a filter.

Aspects of the present invention fulfill these needs and provide further related advantages as described in the following summary.

### SUMMARY OF THE INVENTION

A system for replenishing minerals of dialysate in a dialyzer according to the present invention comprises the technical features as defined in independent claim 1. According to a first aspect of the present disclosure, a hemodialysis system is provided including an arterial blood line for connecting to a patient's artery for collecting blood from a patient, a venous blood line for connecting to a patient's vein for returning blood to a patient, a reusable dialysis machine and a disposable dialyzer.

The arterial blood line and venous blood line may be typical constructions known to those skilled in the art. For example, the arterial blood line may be traditional flexible hollow tubing connected to a needle for collecting blood from a patient's artery. Similarly, the venous blood line may be a traditional flexible tube and needle for returning blood to a patient's vein. Various constructions and surgical procedures may be employed to gain access to a patient's blood including an intravenous catheter, an arteriovenous fistula, or a synthetic graft.

Preferably, the disposable dialyzer has a construction and design known to those skilled in the art including a blood flow path and a dialysate flow path. The term "flow path" is intended to refer to one or more fluid conduits, also referred to as passageways, for transporting fluids. The conduits may be constructing in any manner as can be determined by ones skilled in the art, such as including flexible medical tubing or non-flexible hollow metal or plastic housings. The blood flow path transports blood in a closed loop system by connecting to the arterial blood line and venous blood line for transporting blood from a patient to the dialyzer and back to the patient. Meanwhile, the dialysate flow path transports dialysate in a closed loop system from a supply of dialysate to the dialyzer and back to the dialysate supply. Both the blood flow path and the dialysate flow path pass through the dialyzer, but the flow paths are separated by the dialyzer's semipermeable membrane.

In some embodiments, the hemodialysis system contains a reservoir for storing a dialysate solution. The reservoir connects to the hemodialysis system's dialysate flow path to form a closed loop system for transporting dialysate from the reservoir to the hemodialysis system's dialyzer and back to the reservoir. In some exemplar embodiments, the hemodialysis system possesses two (or more) dialysate reservoirs which can be alternatively placed within the dialysate flow path. In such embodiments, when one reservoir possesses contaminated dialysate, dialysis treatment can continue using the other reservoir while the reservoir with contaminated dialysate is emptied and refilled. The reservoirs may be of any size as required by clinicians to perform an appropriate hemodialysis treatment, or as required to hold accumulated dialysate and excess ultrafiltrate volume removed during an appropriate hemodialysis treatment. However, in some embodiments, the two reservoirs are the same size and are sufficiently small so as to enable the dialysis machine to be easily portable. Some acceptable reservoirs are 0.5 liters to 12.0 liters (L) in size. Other reservoir sizes and volumes may be determined by one skilled in the art.

In some embodiments, the hemodialysis system possesses one or more heaters thermally coupled to the reservoirs for heating dialysate stored within the reservoir(s). In addition, the hemodialysis system can include temperature sensors for measuring the temperature of the dialysate within the reservoir(s). The hemodialysis system can also include one or more fluid mass sensors for detecting the mass of fluid in the reservoir(s). The fluid mass sensor(s) may be any type of sensor for determining the mass of fluid within the reservoir(s). Acceptable fluid mass sensors include resistive strain gauge type sensors, magnetic or mechanical float type sensors, optical interfaces, conductive sensors, ultrasonic sensors, and weight measuring sensors such as a scale or load cell for measuring the weight of the dialysate in the reservoir(s).

In some exemplar embodiments, the hemodialysis system comprises three primary pumps. The first and second "dialysate" pumps are connected to the dialysate flow path for pumping dialysate through the dialysate flow path from a reservoir to the dialyzer and back to the reservoir. In some embodiments, a first pump is positioned in the dialysate flow path "upflow", (meaning prior in the flow path) from the dialyzer while the second pumps is positioned in dialysate flow path "downflow" (meaning subsequent in the flow path) from the dialyzer. In some embodiments, the hemodialysis system's third primary pump is connected to the blood flow path. This third primary pump or "blood" pump pumps blood from a patient through the arterial blood line, through the dialyzer, and through the venous blood line for return to a patient. In exemplar embodiments, the third pump is positioned in the blood flow path, upflow from the dialyzer.

The hemodialysis system can also comprise one or more sorbent filters for removing toxins which have permeated from the blood plasma through the semipermeable membrane into the dialysate. Filter materials for use within the filter are well known to those skilled in the art. For example, suitable materials include resin beds including zirconium based resins. Acceptable materials are also described in U.S. Patent No. 8,647,506 and U.S. Patent Publication No. 2014/0001112. Other acceptable filter materials can be developed and utilized by those skilled in the art without undue experimentation. Depending upon the type of filter material, the filter housing may include a vapor membrane capable of releasing gases such as ammonia.

In a first embodiment, the sorbent filter is connected to the dialysate flow path downflow from the dialyzer so as to remove toxins in the dialysate prior to the dialysate being transported back to a reservoir. In a second embodiment, the filter is outside of the closed loop dialysate flow path, but instead is positioned within a separate closed loop "filter" flow path that selectively connects to either one of the two dialysate reservoirs. In some embodiments, the hemodialysis system includes an additional fluid pump for pumping contaminated dialysate through the filter flow path and its filter.

In some embodiments, the hemodialysis system comprises two additional flow paths in the form of a "drain" flow path and a "fresh dialysate" flow path. The drain flow path can include one or more fluid drain lines for draining the reservoirs of contaminated dialysate, and the fresh dialysate flow path can include one or more fluid fill lines for transporting fresh dialysate from a supply of fresh dialysate to the reservoirs. One or more fluid pumps may be connected to the drain flow path and/or the fresh dialysate flow path to transport the fluids to their intended destination.

In addition, the hemodialysis system can include a plurality of fluid valve assemblies for controlling the flow of blood through the blood flow path, for controlling the flow of dialysate through the dialysate flow path, and for controlling the flow of used dialysate through the filter flow path. The valve assemblies may be of any type of electro-mechanical fluid valve construction as can be determined by one skilled in the art including, but not limited to, traditional electro-mechanical two-way fluid valves and three-way fluid valves. A two-way valve is any type of valve with two ports, including an inlet port and an outlet port, wherein the valve simply permits or obstructs the flow of fluid through a fluid pathway. Conversely, a three-way valve possesses three ports and functions to shut off fluid flow in one fluid pathway while opening fluid flow in another pathway. In addition, the dialysis machine's valve assemblies can include safety pinch valves, such as a pinch valve connected to the venous blood line for selectively permitting or obstructing the flow of blood through the venous blood line. The pinch valve is provided so as to pinch the venous blood line and thereby prevent the flow of blood back to the patient in the event that an unsafe condition has been detected.

According to some embodiments, the hemodialysis system contains sensors for monitoring hemodialysis. To this end, some embodiments of the hemodialysis system comprise at least one flow sensor connected to the dialysate flow path for detecting fluid flow (volumetric and/or velocity) within the dialysate flow path. In addition, some embodiments of the hemodialysis system contain one or more pressure sensors for detecting the pressure within the dialysate flow path, or at least an occlusion sensor for detecting whether the dialysate flow path is blocked. In some embodiments, the dialysis machine also comprises one or more sensors for measuring the pressure and/or fluid flow within the blood flow path. The pressure and flow rate sensors can be separate components, or pressure and flow rate measurements can be made by a single sensor.

Furthermore, some embodiments of the hemodialysis system can include a blood leak detector ("BLD") which monitors the flow of dialysate through the dialysate flow path and detects whether blood has inappropriately diffused through the dialyzer's semipermeable membrane into the dialysate flow path. In some exemplar embodiments, the hemodialysis system comprises a blood leak sensor assembly incorporating a light source which emits light through the dialysate flow path, and a light sensor which receives the light that has been emitted through the dialysate flow path. After passing through the dialysate flow path, the received light is then analyzed to determine if the light has been altered to reflect possible blood in the dialysate.

The hemodialysis system also includes a dialysate-replenishing system for replenishing minerals of the dialysate in the dialyzer. In some embodiments, the dialysate-replenishing system can include: a sorbent filter configured to remove ammonia from the breakdown of urea in dialysate; a first reagent source containing a first reagent solution; a first pump configured to inject the first reagent solution into the dialysate flow path of the sorbent filter; a first mixer coupled to the dialysate flow path and downstream of the first pump; a conductivity sensor configured to measure a level of total dissolved solids of the regenerated dialysis fluid a conductivity sensor configured to measure and level of dissolved solids in the dialysate after the first mixer; and a controller configured to adjust a flow rate of the first reagent solution by adjusting the first pump based at least on the level of dissolved solids in the dialysate. In some embodiments, the conductivity sensor comprises a sodium level sensor configured to measure a a conductivity value of the dialysate and level of sodium in the dialysate after the first mixer, and a controller configured to adjust a flow rate of the first reagent solution by adjusting the first pump based at least on the level of sodium in the dialysate.

The sorbent filter has an outlet that outputs the dialysate to an dialysate flow path. The first mixer is configured to mix the dialysate with the first reagent solution, which can be a solution of sodium carbonate.

The sodium carbonate solution can have a concentration of approximately 1.5 M. The dialysate-replenishing system can also include: a second reagent source containing a second reagent solution can be a solution of a plurality of mineral compounds; and a second pump configured to inject the second reagent solution into the dialysate flow path of the sorbent filter, wherein the second pump is located upstream of the first pump.

The dialysate-replenishing system can also include a second mixer disposed upstream of the first mixer. The second mixer is configured to mix the dialysate with the second reagent solution before first reagent is injected into the dialysate flow path by the first pump. The second reagent solution can be a solution of calcium chloride (CaCl2), magnesium chloride (MgCl2), and potassium acetate (KAc).

The CaCl₂ in the second reagent solution can have a concentration of approximately CaCl₂ 25 - 40 millimolar (mM). In some embodiments, the CaCl₂ concentration is approximately 32.04 mM. The MgCl₂ in the second reagent solution can have a concentration of approximately 12.5 - 20 mM. In some embodiments, the MgCl₂ concentration is approximately 6.02 mM. The KAc in the second reagent solution can have a concentration of approximately 75 - 120 mM. In some embodiments, the Kac concentration is approximately 96.12 mM.

The controller can also adjust a flow rate of the second reagent solution by adjusting the second pump based at least on the level of dissolve solids in the dialysate. Further, the conductivity sensor can be a sodium level sensor configured to measure a conductivity value of the dialysate, and the controlled can be configured to adjust a flow rate of the second reagent solution by adjusting the second pump based at least on the level of sodium in the dialysate.

The hemodialysis system possesses a processor containing the dedicated electronics for controlling the hemodialysis system. The processor contains power management and control electrical circuitry connected to the pump motors, valves, and dialysis machine sensors for controlling proper operation of the hemodialysis system.

The dialysis machine provides a hemodialysis system that is transportable, lightweight, easy to use, patient-friendly and capable of in-home use.

In addition, the hemodialysis system provides an extraordinary amount of control and monitoring not previously provided by hemodialysis systems so as to provide enhanced patient safety.

Other features and advantages of the present invention will be appreciated by those skilled in the art upon reading the detailed description, which follows with reference to the Drawings.

### BRIEF DESCRIPTION OF THE DRAWINGS

FIG. 1 is a flow chart illustrating a first embodiment of the hemodialysis system;
FIG. 2 is the flow chart of FIG. 1 illustrating an embodiment where dialysate avoids the filter by flowing through the bypass flow path;
FIG. 3 is the flow chart of FIG. 1 illustrating an embodiment where dialysate flows through the filter in a closed loop dialysate flow path incorporating a first reservoir;
FIG. 4 is the flow chart of FIG. 1 illustrating an embodiment where dialysate flows through the filter in a closed loop dialysate flow path incorporating a second reservoir;
FIG. 5 is a flow chart illustrating a second embodiment of the hemodialysis system including a closed loop filter flow path which is filtering the fluid in a first reservoir;
FIG. 6 is a flow chart illustrating the second embodiment of the hemodialysis system shown in FIG. 5 wherein the filter flow path which is filtering the fluid in a second reservoir;
FIG. 7A is a flow chart illustrating a hemodialysis system having a system for replenishing dialysate with minerals in accordance with some embodiments according to the invention as claimed;
FIG. 7B is a flow chart illustrating a hemodialysis system having a system for replenishing dialysate with minerals in accordance with some embodiment according to the invention as claimed.
FIG. 8 is a flow chart illustrating a system for replenishing dialysate with minerals in accordance with some embodiments according to the invention as claimed;
FIG. 9 is a chart illustrating results from the system of FIG. 8;
FIG. 10 illustrates a conductivity sensor in accordance with some embodiments;
FIG. 11 illustrates a cross-sectional view of the conductivity sensor shown in FIG. 10; and
FIGS. 12A-C illustrate exemplary electrodes in accordance with some embodiments.

### DETAILED DESCRIPTION OF THE INVENTION

While the present invention is capable of embodiment in various forms, as shown in the drawings, hereinafter will be described the presently preferred embodiments of the invention with the understanding that the present disclosure is to be considered as an exemplification of the invention, which is defined by the appended claims, and it is not intended to limit the invention to the specific embodiments illustrated.

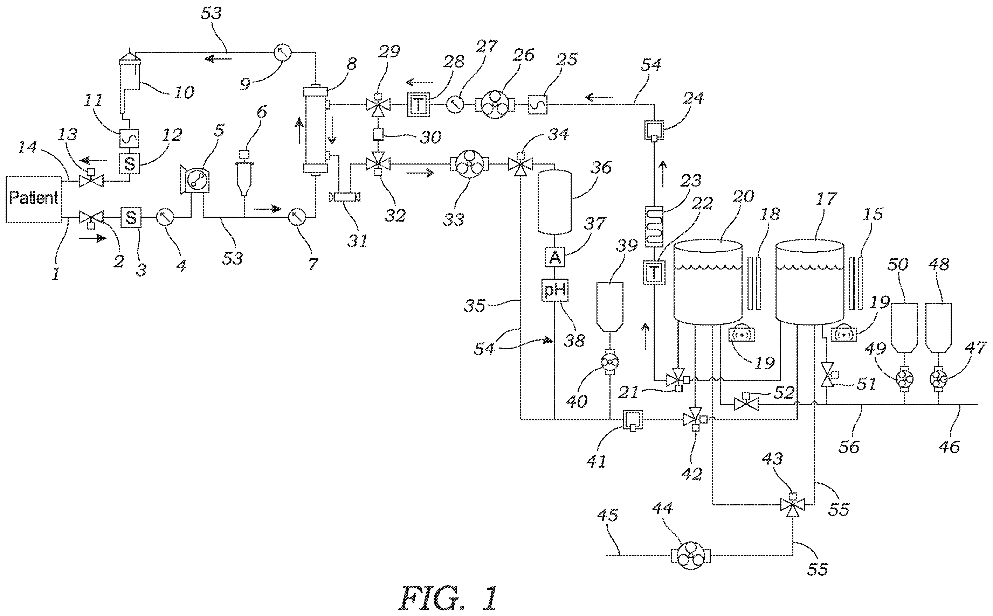

As illustrated in FIGS. 1-7B, the hemodialysis system comprises a blood flow path 53 and a dialysate flow path 54. The hemodialysis system further comprises a reusable dialysis machine and disposable components for performing hemodialysis. The blood flow path 53 includes an arterial blood line 1 for connecting to a patient's artery for collecting blood from a patient, and a venous blood line 14 for connecting to a patient's vein for returning blood to a patient. The arterial blood line 1 and venous blood line 14 may be typical constructions known to those skilled in the art.

The blood flow path 53 transports blood in a closed loop system by connecting to the arterial blood line 1 and venous blood line 14 to a patient for transporting blood from a patient through the dialyzer 8 and back to the patient. In some embodiments, the hemodialysis system comprises a supply of heparin 6 and a heparin pump connected to the blood flow path 53. The heparin pump delivers small volumes of heparin anticoagulant into the blood flow to reduce the risk of blood clotting in the machine. The heparin pump can take the form of a linearly actuated syringe pump, or the heparin pump may be a bag connected with a small peristaltic or infusion pump.

The hemodialysis system further comprises a dialyzer 8 in the dialysate flow path 54 which is of a construction and design known to those skilled in the art. Preferably, the dialyzer 8 includes a large number of hollow fibers which form a semipermeable membrane. Suitable dialyzers can be obtained from Fresenius Medical Care, Baxter International, Inc., Nipro Medical Corporation, and other manufacturers of hollow fiber dialyzers. Both the blood flow path 53 and dialysate flow path 54 travel through the dialyzer 8 which comprises an inlet for receiving dialysate, an outlet for expelling dialysate, an inlet for receiving blood from a patient, and an outlet for returning blood to a patient. Preferably, the dialysate flows in the opposite direction to the blood flowing through the dialyzer 8 with the dialysate flow path 54 isolated from the blood flow path 53 by a semipermeable membrane (not shown).

As explained in greater detail below, the dialysate flow path 54 transports dialysate in a closed loop system in which dialysate is pumped from a reservoir (17 or 20) to the dialyzer 8 and back to the reservoir (17 or 20). Both the blood flow path 53 and the dialysate flow path 54 pass through the dialyzer 8, but are separated by the dialyzer's 8 semipermeable membrane.

In some embodiments, the hemodialysis system includes three primary pumps (5, 26 & 33) for pumping blood and dialysate. For purposes herein, the term "pump" is meant to refer to both the pump actuator which uses suction or pressure to move a fluid, and the pump motor for mechanically moving the actuator. Suitable pump actuators may include an impeller, piston, diaphragm, the lobes of a lobe pump, screws of a screw pump, rollers or linear moving fingers of a peristaltic pump, or any other mechanical construction for moving fluid as can be determined by those skilled in the art. Meanwhile, the pump's (5, 26, or 33) motor is the electromechanical apparatus for moving the actuator. The motor may be connected to the pump actuator by shafts or the like. In an exemplar embodiment, the dialysate and/or blood flow through traditional flexible tubing and each of the pump actuators consist of a peristaltic pump mechanism wherein each pump actuator includes a rotor with a number of cams attached to the external circumference of the rotor in the form of "rollers", "shoes", "wipers", or "lobes" which compress the flexible tube. As the rotor turns, the part of the tube under compression is pinched closed (or "occludes") forcing the fluid to be pumped through the tube. Additionally, as the tube opens to its natural state after the passing of the cam fluid flow is induced through the tube.

The first and second primary pumps (26 and 33) are connected to the dialysate flow path 54 for pumping dialysate through the dialysate flow path 54 from the reservoir (17 or 20) to the dialyzer 8 and back to the reservoir (17 or 20). A first pump 26 is connected to the dialysate flow path 54 "upstream," (meaning prior in the flow path) from the dialyzer 8 while the second pump 33 is connected to the dialysate flow path 54 "downstream" (meaning subsequent in the flow path) from the dialyzer 8. Meanwhile, the hemodialysis system's third primary pump 5 is connected to the blood flow path 53. The third primary pump 5, also referred to as the blood pump, pumps blood from a patient through the arterial blood line 1, through the dialyzer 8, and through the venous blood line 14 for return to a patient. It is preferred that the third primary pump 5 be connected to the blood flow path 53 upstream from the dialyzer 8.

The hemodialysis system can contain more or less than three primary pumps. For example, the dialysate may be pumped through the dialyzer 8 utilizing only a single pump. However, in some preferred embodiments, the hemodialysis system contain two pumps. In these embodiments, it is even more preferred that the hemodialysis system contains a first pump 26 upstream from the dialyzer 8 and a second pump 33 downflow from the dialyzer 8.

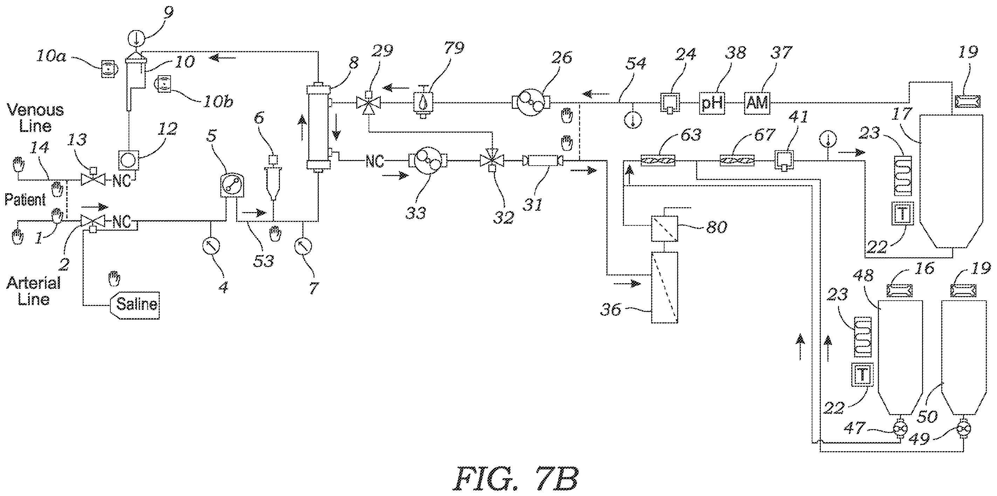

In some embodiments, such as those illustrated in FIGS. 1-6, the hemodialysis system can have two or more reservoirs (17 and 20) for storing dialysate solution. Alternatively, and as illustrated in FIG. 7B, the hemodialysis system can have one reservoir 17 for storing dialysate solution.

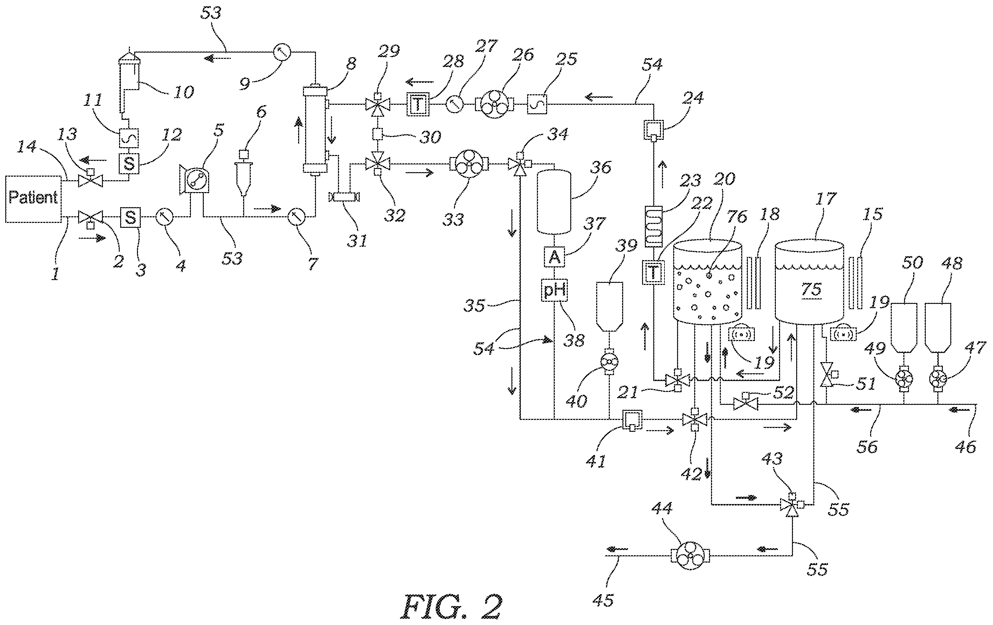

Both of the reservoirs (17 and 20) may be connected simultaneously to the dialysate flow path 54 to form one large source of dialysate. However, this is not considered preferred. Instead, in some embodiments, the hemodialysis system comprises a valve assembly 21 for introducing either, but not both, of the two reservoirs (17 or 20) into the dialysate flow path 54 to form a closed loop system for transporting a dialysate from one of the two reservoirs (17 or 20) to the dialyzer 8 and back to that same reservoir (17 or 20). After the dialysate in the first reservoir 17 has been used, is no longer sufficiently clean, or does not possess appropriate chemical properties, the hemodialysis system's valve 21 is controlled to remove the first reservoir 17 from the dialysate flow path 54 and substitute the second reservoir 20, which has fresh dialysate 75, into the dialysate flow path 54. Thus, when one reservoir (17 or 20) possesses contaminated dialysate 76 (as shown in FIGS. 2-6), and that reservoir (17 or 20) needs to be emptied and refilled with freshly generated dialysis fluid 75, dialysis treatment can continue using the other reservoir (17 or 20).

In this manner, the hemodialysis system may switch between each reservoir 17 and 20 multiple times over the course of a treatment. Furthermore, the presence of two reservoirs (17 and 20) as opposed to one reservoir allows for the measurement of the flow rate for pump calibration or ultrafiltration measurement, while isolating the other reservoir (17 or 20) while it is being drained or filled. Though the reservoirs (17 and 20) may be of any size as required to hold accumulated dialysate and excess ultrafiltrate volume removed during an appropriate hemodialysis treatment, some preferred reservoir(s) have a total volume between 8 L and 12 L.

As illustrated in FIGS. 1-7B, the hemodialysis system also comprises a sorbent filter 36 (also referred to herein as a "filter") connected to the dialysate flow path 54 for removing toxins which have permeated from the blood plasma through the semipermeable membrane into the dialysate. In a first embodiment, the filter 36 is connected to the dialysate flow path 54 downstream from the dialyzer 8 so as to remove toxins transferred by the dialyzer 8 into the dialysate prior to the dialysate being transported to the reservoir (17 or 20). Filter 36 materials for use with the dialysis machine are well known to those skilled in the art. For example, suitable materials include resin beds including zirconium based resins. Preferably, the filter 36 comprises a housing containing layers of zirconium oxide, zirconium phosphate, urease, and carbon. Acceptable materials are described in U.S. Patent No. 8,647,506 and U.S. Patent Application Publication No. 2014/0001112. Other acceptable filter 36 materials can be developed and utilized by those skilled in the art without undue experimentation.

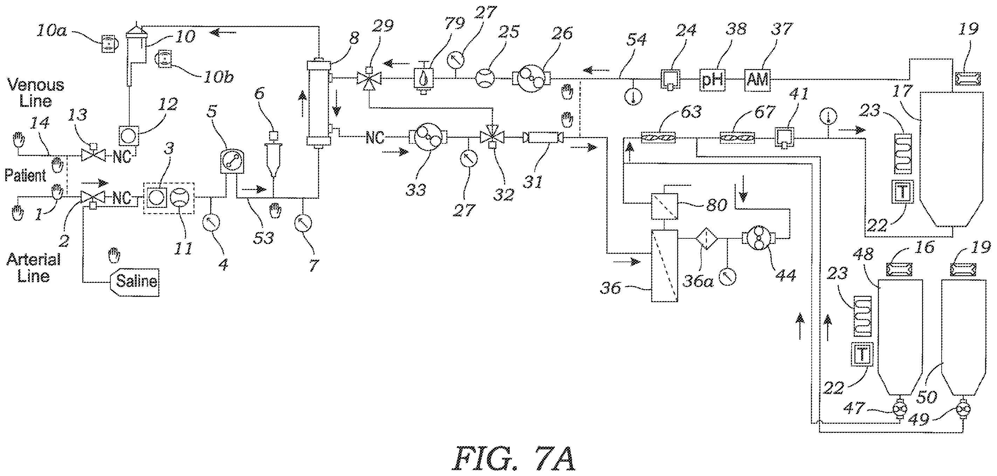

The filter's 36 housing may or may not include a degassing membrane 80 capable of releasing gases including air and carbon dioxide, but not liquids, and particularly not the dialysate liquid flowing through the filter. For example, in the embodiment illustrated in FIGS. 7A & 7B, the dialysate flow path 54 includes a degasser 80 positioned downstream of the sorbent filter 36. The sorbent filter 36, in turn, has an air inlet having a filter 36a, pressure sensor, and pump 44. Sorbent regeneration degassing may be accomplished by introducing a stream of air through the air inlet, which is substantially free of CO₂, into the regenerated dialysate. Preferably, the pump 44 introduces the stream of air into the sorbent filter 36 at about the same approximate flowrate as the flowrate of the liquid through the dialysate flow path. The combined air-liquid fluid may then be exposed to a hydrophobic membrane within the degasser 80 where the gas is free to exit the system, but liquid continues to flow through the dialysate flow path.

In some embodiments, dialyzer 8 further comprises a sorbent dialysis device (not shown). In the sorbent dialysis device, ammonia in the dialysate is generated by a reaction of urea with urease. The ammonia in equilibrium with ammonium is adsorbed by an ion exchange material. After some time, the capacity of the ion exchange material for ammonium is used up and ammonia and/or ammonium start to leach out. As such, a dialysate quality sensor 700 (not illustrated) is required in order to detect whether an unsafe amount of ammonia is present in the dialysate due to leaching from the sorbent dialysis device. In some embodiments, the dialysis flow path 54 can include one or more dialysate quality sensors 700, such as an ammonium sensor 37 and/or a pH sensor 38. In some embodiments, the dialysis flow path 54 comprises an ammonium sensor 37 and a pH sensor 38, both of which can be located immediately downstream of the sorbent filter 36 (best illustrated in FIGS. 1-6). When the sorbent filter 36 has been exhausted, the filter 36 may begin to release ammonium ions as a result of the filtering chemical reaction. At certain levels, ammonium ions in the dialysis fluid can harm the patient. Preferably, the ammonium sensor 37 measures the quantity of ammonium ions in parts per million (ppm). In some embodiments, when the measurement reaches a range of approximately 1 ppm to 20 ppm, a warning state will be activated, and treatment with this dialysate can be automatically stopped.

Alternatively, when the ppm of ammonium ions passes above a certain ppm threshold (e.g., 5 ppm, 10 ppm), the dialysis fluid can be drained, and dialysis treatment may continue by using fresh dialysate 75 using the alternative reservoir (17 or 20). Similarly, the pH sensor 38 also acts as a safety feature and supports the measurement of ammonium ions. As the pH of the dialysis fluid changes, the equilibrium state of ammonia (NH3) and ammonium ions (NH4+) can change. In some embodiments, if the pH of the dialysis fluid is measured to be outside the range of approximately 6.4 to 7.8 pH, a warning state can be activated, and the dialysis treatment can be ended.

As illustrated in FIGS. 1-6, some embodiments of the hemodialysis system comprise a reagent bag 39 and reagent pump 40 for introducing reagents into the dialysate flow path 54 immediately after the sorbent filter 36. The reagent bag 39 holds a concentrated solution of salts and ions to reinfuse the filter dialysis fluid. The conductivity sensor 41 can be a sodium level sensor configured to measure the total dissolved solids of the regenerated dialysis fluid. Through the action of filtering waste, the sorbent filter 36 also removes beneficial ions from the dialysis fluid, such as calcium and salt. Before the filtered dialysis fluid can be recirculated, it must be reinfused with calcium and salts so that the dialysis fluid does not draw these beneficial ions from the patient's blood. Preferably, the reagent bag 39 will hold between 1 and 3 liters of concentrated reagent. The reagent pump 40 can be any type of pump such as a peristaltic pump or diaphragm pump. To ensure that the hemodialysis system is introducing the proper amount of salts and ions into the dialysate, a conductivity sensor 41 may be positioned within the dialysate flow path 54 immediately after the reagent bag 39. In this way, the conductivity sensor 41 serves as a safety feature, measuring the total dissolved solids of the regenerated dialysis fluid. In the event that the total dissolved solids are detected to not be within a prescribed range, the operation of the reagent pump 40 can be increased or decreased, or alternatively, treatment can be stopped entirely. For example, if a fault state is detected in the dialysis fluid, then the fluid can be redirected by 3-way valves 29 and 32 through the dialyzer bypass path 30 so that dialysate does not meet the patient's blood in the dialyzer 8. More specifically, the 3-way valve 29 directs dialysis fluid to the dialyzer's 8 inlet and the 3-way valve 32 directs dialysate from the dialyzer's 8 outlet back through the dialysate flow path 54. However, if a fault state is detected in the dialysis fluid, such as the temperature being too low or excessive ammonium ions are detected in the dialysate, then the dialysis fluid is redirected by 3-way valves 29 and 32 to bypass the dialyzer 8, through dialyzer bypass path 30.

In some embodiments, and as illustrated in FIGS. 1-4, the hemodialysis system further comprises a drain flow path 55 to dispose of waste dialysate from the reservoirs (17 and 20). In the embodiment illustrated in the FIGS 1 - 4, the drain flow path 55 is connected to both reservoirs (17 and 20). Waste dialysate may drain through the drain flow path 55 through a gravity feed, or the hemodialysis system may include a pump 44 of any type as can be selected by those skilled in the art to pump used dialysate to be discarded, such as to a traditional building sewer line 45.

For the embodiment illustrated in FIGS. 1 - 4, the hemodialysis system can include a source 46 of dialysate fluid to replenish each of the reservoirs (17 and 20). Preferably, the source of dialysate fluid includes a supply of clean water 46 that is mixed with concentrated reagents to provide dialysate of desired properties. In a preferred embodiment, the supply of clean water 46 is provided by a reverse osmosis ("RO") machine located adjacent to the device which produces clean water and then adds chemical concentrates to create the dialysate fluid. The fluid is supplied through a "fresh dialysate" flow path 56 to the reservoirs (17 and 20). In some preferred embodiments, the hemodialysis system comprises a source of concentrated reagents which can be stored in disposable bags. Preferably, the concentrated reagents contain one or more of the following: bicarbonate solution, acid solution, lactate solution, salt solution. It is necessary to separate some of the reagents into two bags (48 and 50) to prevent undesirable interactions or precipitation of solutes. The concentrated reagents sources (48 and 50) are connected by reagent pumps (47 and 49) to the supply line 46. The activation of the reagent pumps (47 and 49) introduces the concentrated reagents into the supply of water to provide dialysate to the reservoirs (17 and 20).

Still with reference to FIGS. 1-4, as an alternative to using the sorbent filter 36, the hemodialysis system can include a supplemental "bypass" flow path 35 that selectively transports dialysis around the sorbent filter 36. The bypass flow path 35 includes a 3-way valve 34 upstream of the sorbent filter 36. In this way, the 3-way valve 34 is switched to direct the dialysis fluid through sorbent filter 36, or alternatively, the 3-way valve 34 is switched to direct dialysate through the bypass flow path 35 to avoid the sorbent filter 36. For example, if a sorbent filter 36 is not available, or if the sorbent filter 36 has become spent, or if a sorbent filter 36 is not required or preferred for a particular patient treatment, then the 3-way valve 34 is switched to direct the dialysis fluid down the bypass flow path 35.

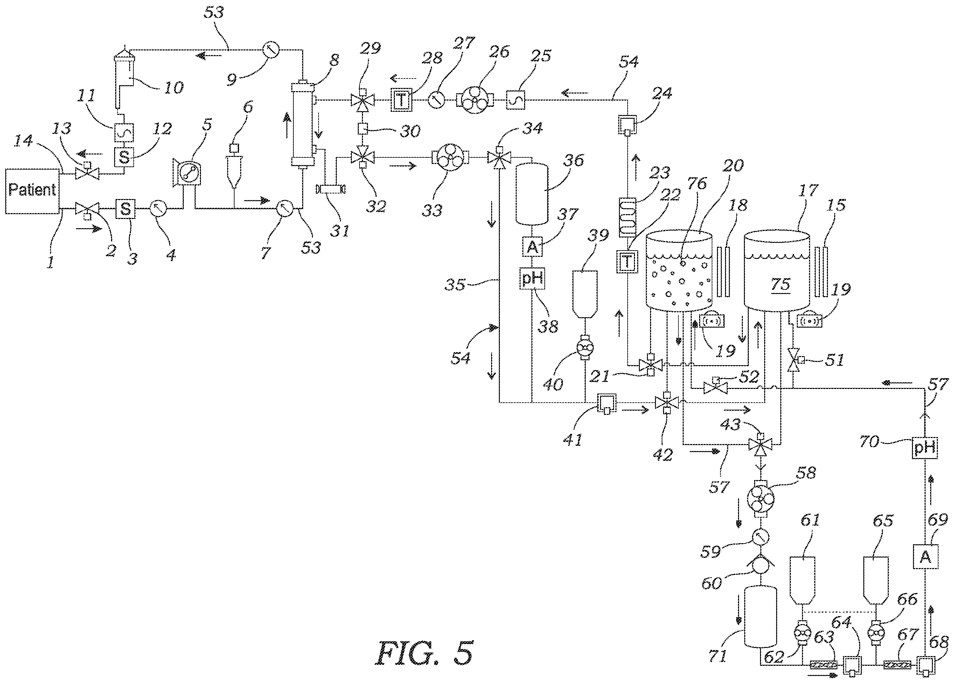

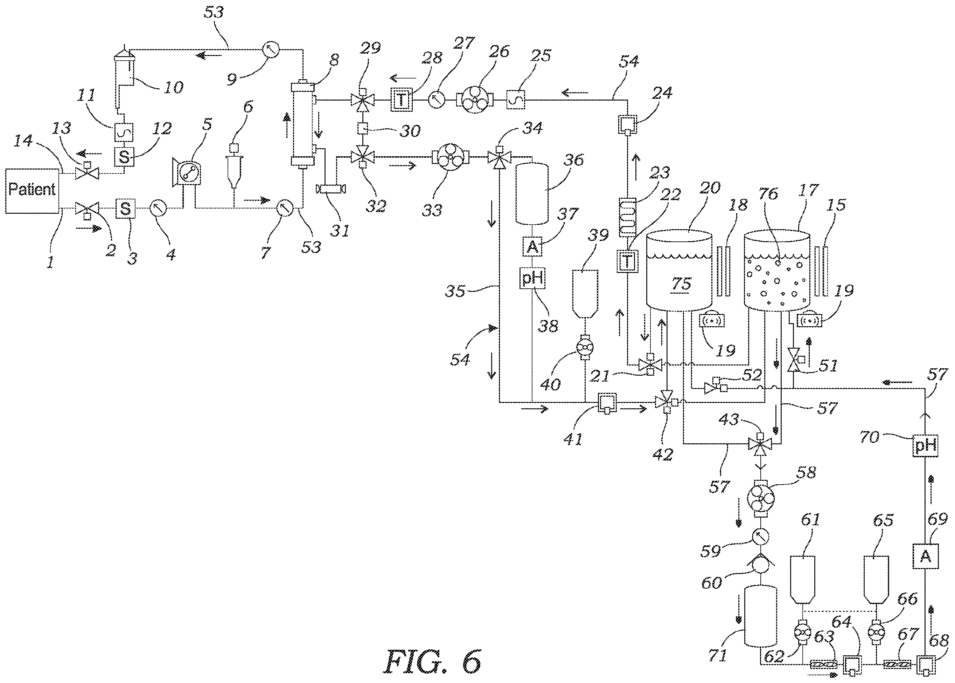

In an alternative embodiment, and as illustrated in FIGS. 5 and 6, a sorbent filter 71 is located outside of the closed loop dialysate flow path 54. The hemodialysis system includes a separate closed loop "filter" flow path 57 that selectively connects to either one of the two dialysate reservoirs (17 or 20), and the sorbent filter 71 is positioned in-series in the closed loop filter flow path 57. Preferably, the dialysis machine includes an additional fluid pump 58 for pumping contaminated dialysate through the filter flow path 57 and the sorbent filter 71. As illustrated in FIGS. 5 and 6, some embodiments comprise a filter flow path 57 having a 3-way valve 43 which determines which reservoir (17 or 20) is drained of contaminated dialysate. For example, FIG. 5 illustrates the 3-way valve 43 connecting reservoir 20, but not reservoir 17, to the filter flow path 57. Further, FIG. 6 illustrates the 3-way valve 43 connecting reservoir 17, but not reservoir 20, to the filter flow path 57. The filter flow path 57 may include a pump 58, or the dialysate may dispense contaminated dialysate from reservoirs (17 or 20) through a gravity feed. In addition, preferably the filter flow path 57 includes a pressure sensor 59, a check valve 60, an ammonium sensor 69, and a pH sensor 70.

This embodiment of the hemodialysis machine also includes a system for introducing reagents into the filter flow path 57. As illustrated in FIGS. 5 and 6, the filter flow path 57 includes a first reagent source 61, preferably containing salts, and a second reagent source 65, preferably containing bicarbonate and lactate solution. These reagents are introduced into the filter flow path 57 using pumps (62 and 66), and mixers (63 and 67). Preferably the filter flow path 57 also possesses safety features in the form of (1) an ammonium sensor 69 to ensure that the filter 71 is not spent and/or introducing unacceptable ammonium ions into the dialysate; (2) a pH sensor 70 to support the measurement of ammonium ions and detect pH within the dialysate; and (3) conductivity sensors (64 and 68) which monitor whether the reagents have been properly introduced into the cleaned dialysate to provide the proper amounts of beneficial ions. Finally, the filter flow path 57 comprises a pair of check valves (51 and 52) which open and close to ensure that the now cleaned dialysate is returned to the reservoir (17 or 20) from which the contaminated dialysate had been drained from.

In some embodiments, and as illustrated in FIGS. 1-7B, the hemodialysis system can comprise a heater 23 thermally connected to the dialysate flow path 54 or to reservoirs (17 and/or 20) for heating the dialysate to a desired temperature. For example, in the embodiments illustrated in FIGS. 1 - 6, a single heater 23 is thermally coupled to the dialysate flow path 54 downstream of both reservoirs (17 and 20). However, the hemodialysis may include additional heaters 23, and the one or more heaters 23 may be in different locations. For example, in an alternative embodiment, the hemodialysis system includes two heaters 23, with a single heater 23 thermally coupled to each reservoir (17 and 20). The one or more heaters 23 are preferably activated by electricity and includes a resistor which produces heat with the passage of an electric current.

In addition, the various embodiments of the hemodialysis system described herein can possess various sensors for monitoring hemodialysis, and in particular, the dialysate flow path 54 and blood flow path 53. To this end, some embodiments of the hemodialysis system can comprise one or more flow sensors 25 connected to the dialysate flow path 54 for detecting fluid flow (volumetric and/or velocity) within the dialysate flow path 54. In other embodiments, the hemodialysis system does not comprise a flow sensor 25. In addition, some hemodialysis system embodiments comprise one or more pressure, or occlusion, sensors (27) for detecting the pressure within the dialysate flow path 54. Additionally, some embodiments of the hemodialysis system can comprise one or more sensors for measuring the pressure (4, 7, and 9) with or without fluid flow 11 within the blood flow path 53.

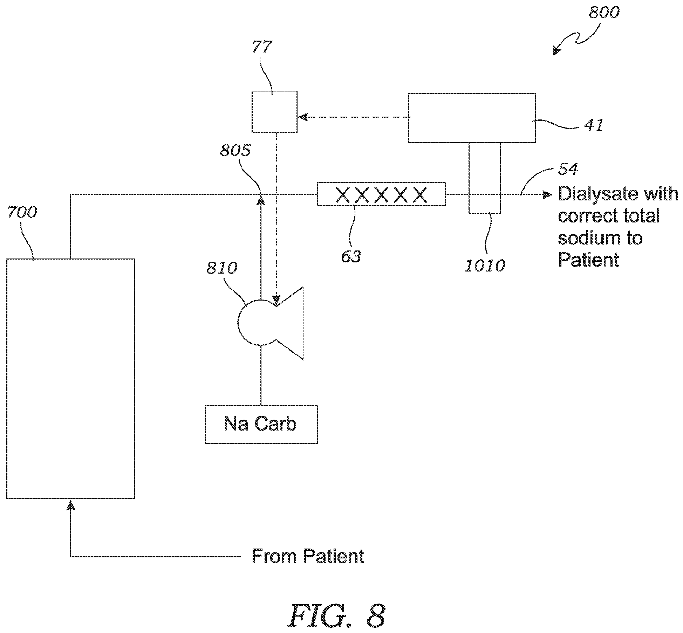

In some embodiments, the hemodialysis system comprises temperature sensors (15, 22 24, and 28) for measuring the temperature of the dialysate throughout the dialysate flow path 54. In addition, the hemodialysis system can comprise fluid mass sensors for detecting the mass of fluid in the reservoirs (17 and 20). Further, some embodiments of the fluid mass sensors can include either capacitive fluid mass sensors (15 and 18) such as those described in U.S. Patent No. 9,649,419, or ultrasonic fluid level sensors. In some embodiments, the weight, and therefore level of dialysate, of each reservoir (17 and 20) is measured by a strain gauge sensor (16 or 19) connected to a processor 77 (shown in FIG. 8, and described in further detail below).

In some embodiments, and as illustrated in FIG. 7B, the hemodialysis system does not comprise a bubble sensor 3 in the arterial line, a flow sensor 11 in the blood circuit, the dialysate flow sensor 25 in the dialysis circuit, and pressure sensor 27 in the dialysis circuit.

Furthermore, in some embodiments, and as illustrated in FIGS. 1-7B, the hemodialysis system can include a blood leak detector 31 which monitors the flow of dialysate through the dialysate flow path 54 and detects whether blood has inappropriately diffused through the dialyzer's 8 semipermeable membrane into the dialysate flow path 54.

Preferably, the hemodialysis system also contains a first pinch valve 2 connected to the arterial blood line 1 for selectively permitting or obstructing the flow of blood through the arterial blood line 1, and a second pinch valve 13 connected to the venous blood line 14 for selectively permitting or obstructing the flow of blood through the venous blood line 14. The pinch valves (2 and 13) are provided so as to pinch the arterial blood line 1 and venous blood line 14, respectively, to prevent the flow of blood back to the patient in the event that any of the sensors have detected an unsafe condition. Providing still additional safety features, the hemodialysis system includes blood line bubble sensors (3 and 12) to detect if an air bubble travels backwards down the arterial line 1 (blood leak sensor 3) or venous line 14 (blood leak sensor 12). Further, the blood flow path 53 may include a bubble trap 10 which has a pocket of pressurized air inside a plastic housing. Bubbles rise to the top of the bubble trap 10, while blood continues to flow to the lower outlet of the bubble trap 10. This component reduces the risk of bubbles traveling into the patient's blood.

To control the flow and direction of blood and dialysate through the hemodialysis system, the hemodialysis system includes a variety of fluid valves for controlling the flow of fluid through the various flow paths of the hemodialysis system. The various valves include pinch valves and 2-way valves which must be opened or closed, and 3-way valves which divert dialysate through a desired flow pathway as intended. In addition to the valves identified above, some embodiments of the hemodialysis system comprise a 3-way valve 21 located at the reservoirs' (17 and 20) outlets which determines from which reservoir (17 or 20) dialysate passes through the dialyzer 8. An additional 3-way valve 42 determines to which reservoir (17 or 20) the used dialysate is sent to. Finally, 2-way valves (51 and 52), which may be pinch valves, are located at the reservoirs' (17 and 20) inlets to permit or obstruct the supply of fresh dialysate to the reservoirs (17 and 20). Of course, alternative valves may be employed as can be determined by those skilled in the art, and the present invention is not intended to be limited the specific 2-way valve or 3-way valve that has been identified.

In addition, the hemodialysis system includes a processor 77 (illustrated in FIG. 8) and a user interface (not shown). The processor 77 contains the dedicated electronics for controlling the hemodialysis system including the hardware and software, and power management circuitry connected to the pump motors, sensors (including reservoir mass strain gauge sensor(s) (16 and/or 19), blood leak sensor 31, ammonia sensor 37, pressure and flow rate sensors (4, 7, 9, 11, 25, 27, and 59), temperature sensors (22, 24 and 28), blood line bubble sensors (3 and 12), valves (2, 13, 21, 29, 32, 34, 42, 43, 51, 52, and 60), and heater 23 for controlling proper operation of the hemodialysis system. The processor 77 monitors each of the various sensors (3, 4, 7, 9, 11, 12, 15, 16, 18, 19, 22, 24, 25, 27, 28, 31, 37, and 59) to ensure that hemodialysis treatment is proceeding in accordance with a preprogrammed procedure input by medical personnel into the user interface. The processor 77 can be a general-purpose computer or microprocessor including hardware and software as can be determined by those skilled in the art to monitor the various sensors (3, 4, 7, 9, 11, 12, 15, 16, 18, 19, 22, 24, 25, 27, 28, 31, 37, and 59) and provide automated or directed control of the heater 23, pumps (5, 6, 26, 33, 40, 44, 47 and 49), and pinch valves (2 and 13). The processor 77 can be located within the electronics of a circuit board or within the aggregate processing of multiple circuit boards and memory cards.

Also not shown, the hemodialysis system includes a power supply for providing power to the processor 77, user interface, pump motors, valves (2, 13, 21, 29, 32, 34, 42, 43, 51, 52, and 60) and sensors (3, 4, 7, 9, 11, 12, 15, 16, 18, 19, 22, 24, 25, 27, 28, 31, and 37). The processor 77 can also be connected to the dialysis machine sensors (3, 4, 7, 9, 11, 12, 15, 16, 18, 19, 22, 24, 25, 27, 28, 31, 37, and 59), and pinch valves (2 and 13) by traditional electrical circuitry.

In operation, the processor 77 is electrically connected to the first, second and third primary pumps (5, 26, and 33) for controlling the activation and rotational velocity of the pump motors, which in turn controls the pump actuators, which in turn controls the pressure and fluid velocity of blood through the blood flow path 53 and the pressure and fluid velocity of dialysate through the dialysate flow path 54. By independently controlling operation of the dialysate pumps 26 and 33, the processor 77 can maintain, increase or decrease the pressure and/or fluid flow within the dialysate flow path within the dialyzer 8. Moreover, by controlling all three pumps (5, 26, and 33) independently, the processor 77 can control the pressure differential across the dialyzer's 8 semipermeable membrane to maintain a predetermined pressure differential (zero, positive or negative), or maintain a predetermined pressure range. For example, most hemodialysis is performed with a zero or near zero pressure differential across the semipermeable membrane, and to this end, the processor 77 can monitor and control the pumps (5, 26, and 33) to maintain this desired zero or near zero pressure differential. Alternatively, the processor 77 can monitor the pressure sensors (4, 7, 9, 27, and 59) and control the pump motors, and in turn pump actuators, to increase and maintain positive pressure in the blood flow path 53 within the dialyzer 8 relative to the pressure of the dialysate flow path 54 within the dialyzer 8. Advantageously, this pressure differential can be affected by the processor to provide ultrafiltration and the transfer of free water and dissolved solutes from the blood to the dialysate.

In some embodiments, the processor 77 monitors the blood flow sensor 11 to control the blood pump 5 flowrate. It uses the dialysate flow sensor 25 to control the dialysate flow rate from the upstream dialysate pump 26. The processor 77 then uses the mass strain gauge sensor(s) (16 and/or 19) to control the flowrate from the downstream dialysate pump 33. The change in fluid level (or volume) in the dialysate reservoir (17 or 20) is identical to the change in volume of the patient. By monitoring and controlling the level in the reservoir (17 or 20), forward, reverse, or zero ultrafiltration can be accomplished.

Moreover, the processor 77 monitors all of the various sensors (3, 4, 7, 9, 11, 12, 15, 16, 18, 19, 22, 24, 25, 27, 28, 31, 37, and 59) to ensure that the hemodialysis machine is operating efficiently and safely, and in the event that an unsafe or non-specified condition is detected, the processor 77 corrects the deficiency or ceases further hemodialysis treatment. For example, if the venous blood line 14 pressure sensor 9 indicates an unsafe pressure or the bubble sensor 12 detects a gaseous bubble in the venous blood line 14, the processor 77 signals an alarm, the pumps are deactivated (5, 6, 26, 33, 40, 44, 47 and 49), and the pinch valves (2 and 13) are closed to prevent further blood flow back to the patient. Similarly, if the blood leak sensor 31 detects that blood has permeated the dialyzer's 8 semipermeable membrane, the processor 77 signals an alarm and ceases further hemodialysis treatment.

The dialysis machine's user interface may include a keyboard or touch screen (not shown) for enabling a patient or medical personnel to input commands concerning treatment or enable a patient or medical personnel to monitor performance of the hemodialysis system. Moreover, the processor 77 can include Wi-Fi or Bluetooth connectivity for the transfer of information or control to a remote location.

Hereinafter will be identified the various components of the preferred hemodialysis system with the numbers corresponding to the components illustrated in the Figures.

| | |
|---|---|
| **1** | Arterial tubing connection |
| **2** | Pinch valve, arterial line. Used to shut off the flow connection with the patient, in case of an identified warning state potentially harmful to the patient. |
| **3** | Bubble sensor, arterial line |
| **4** | Pressure sensor, blood pump inlet |
| **5** | Blood pump |
| **6** | Heparin supply and pump |
| **7** | Pressure sensor, dialyzer input |
| **8** | Dialyzer |
| **9** | Pressure sensor, dialyzer output |
| **10** | Bubble trap |
| **11** | Flow sensor, blood Circuit |
| **12** | Bubble sensor, venous line |
| **13** | Pinch valve, venous line |
| **14** | Venous tubing connection |
| **15** | Primary fluid mass sensor, first reservoir |
| **16** | Mass strain gauge sensor, second reservoir |
| **17** | First reservoir which holds dialysis fluid |
| **18** | Primary fluid mass sensor, second reservoir |
| **19** | Mass strain gauge sensor, first reservoir |
| **20** | Second reservoir which holds dialysis fluid |
| **21** | 3-way valve, reservoir outlet. |
| **22** | Temperature sensor, heater inlet. |
| **23** | Fluid heater for heating the dialysis fluid from approximately room temperature or tap temperature, up to the human body temperature of 37 °C. |
| **24** | Combined conductivity and temperature sensor |
| **25** | Flow sensor, Dialysis Circuit |
| **26** | Dialysis pump, dialyzer inlet |
| **27** | Pressure sensor, Dialysis Circuit |
| **28** | Temperature sensor, dialyzer inlet |
| **29** | 3-way valve, dialyzer inlet |
| **30** | Bypass path, dialyzer |
| **31** | Blood leak detector |
| **32** | 3-way valve, dialyzer outlet |
| **33** | Dialysis pump, dialyzer outlet |
| **34** | 3-way valve, sorbent filter bypass |
| **35** | Sorbent filter bypass path |
| **36** | Sorbent filter |
| **37** | Ammonium ion sensor. |
| **38** | pH sensor |
| **39** | Reagent bag holds a concentrated solution of salts and ions |
| **40** | Pump, sorbent filter reinfusion. |
| **41** | Combined conductivity and temperature sensor, sorbent filter outlet. |
| **42** | 3-way valve, reservoir recirculation. |
| **43** | 3-way valve, reservoir drain. |
| **44** | Pump, reservoir drain. |
| **45** | Drain line connection. |
| **46** | Fresh dialysate supply |
| **47** | Pump which delivers concentrated reagents from reagent bag into fresh dialysate flow path |
| **48** | Reagent bag which holds a concentrated reagent that is introduced into fresh dialysate flow path. |
| **49** | Pump which delivers concentrated reagents from reagent bag into the water line. |
| **50** | Reagent bag which holds a concentrated reagent that will be mixed with water to form dialysis fluid. |
| **51** | Pinch valve, first reservoir inlet. |
| **52** | Pinch valve, second reservoir inlet. |
| **53** | Blood flow path |
| **54** | Dialysate flow path |
| **55** | Drain flow path |
| **56** | Fresh dialysis flow path |
| **57** | Filter flow path |
| **58** | Pump, filter flow path |
| **59** | Pressure sensor, filter flow path |
| **60** | Check valve |
| **61** | Reagents - salts |
| **62** | Pump, reagents |
| **63** | Mixer |
| **64** | Conductivity tester |
| **65** | Reagents - bicarbonate / lactate |
| **66** | Pump, reagents |
| **67** | Mixer |
| **68** | Conductivity tester |
| **69** | Ammonium ion sensor |
| **70** | pH sensor |
| **71** | Sorbent filter |
| **75** | Fresh dialysate |
| **76** | Contaminated dialysate |
| **77** | Processor |
| **80** | Degasser |

### Treatment Options

The hemodialysis system provides increased flexibility of treatment options based on the required frequency of dialysis, the characteristics of the patient, the availability of dialysate or water and the desired portability of the dialysis machine. For all treatments, the blood flow path 53 transports blood in a closed loop system by connecting to the arterial blood line 1 and venous blood line 14 to a patient for transporting blood from a patient to the dialyzer 8 and back to the patient.

With reference to FIG. 2, a first method of using the hemodialysis system does not require the use of a sorbent filter 36. Water is introduced to the machine through the fresh dialysate flow path 56 from a water supply 46 such as water supplied through RO. If needed, chemical concentrates are added to the clean water using the chemical concentrate pumps 47 and 49. The mixed dialysate is then introduced to reservoirs (17 and 20). For this treatment, the fresh dialysate 75 from a first reservoir (17 or 20) is recirculated past the dialyzer 8 through sorbent filter bypass path 35 back to the same reservoir (17 or 20). When the volume of the reservoir (17 or 20) has been recirculated once, the reservoir (17 or 20) is emptied through the drain flow path 55 and the reservoir (17 or 20) is refilled through the fresh dialysate flow path 56.

Meanwhile, while the first reservoir (17 or 20) is being emptied and refilled, hemodialysis treatment continues using the second reservoir (17 or 20). For example, and as illustrated in FIG. 2, once the processor 77 (shown in FIG. 8) has determined that all dialysate has recirculated once, or determined that the dialysate is contaminated, the processor 77 switches all pertinent valves (21, 42, 43, 51 and 52) to remove the first reservoir 20 from patient treatment, and inserts the second reservoir 17 into the dialysate flow path 54. The fresh dialysate 75 from the second reservoir 17 is recirculated past the dialyzer 8 through sorbent filter bypass path 35 and back to the same reservoir 17. This switching back and forth between reservoirs (17 and 20) continues until the dialysis treatment is complete. This operation is similar, but not the same, as traditional single-pass systems because no sorbent filter 36 is used.

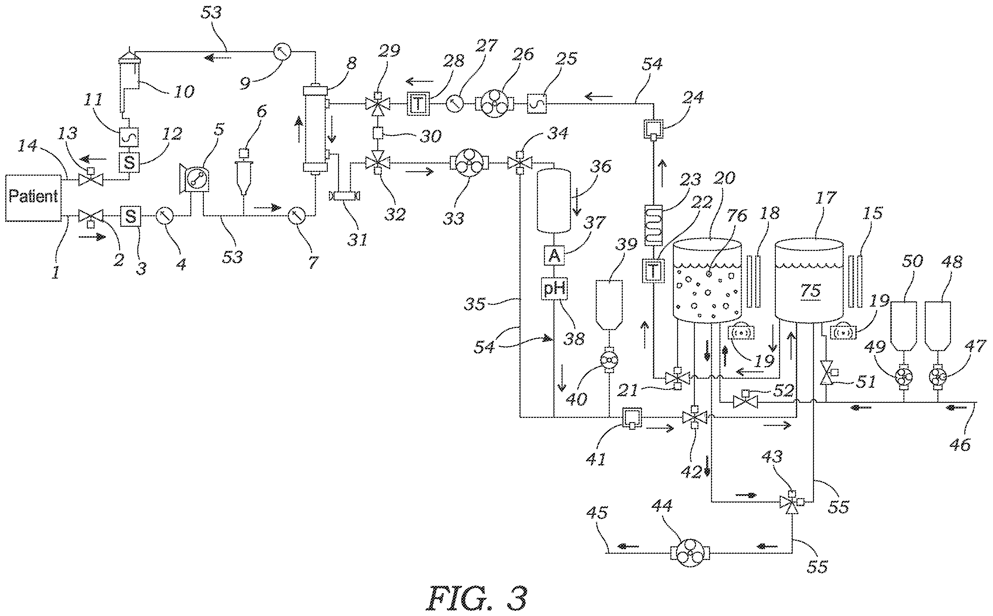

Alternatively, and as illustrated in FIG. 3, the sorbent filter 36 filters the dialysate after it has passed through the dialyzer 8. To this end, the processor 77 switches the 3-way valve 34 to incorporate the sorbent filter 36 into the dialysate flow path 54, and the processor 77 switches the various valve assemblies (21, 42, 43, 51 and 52) to utilize reservoir 17 during dialysis treatment. Fresh dialysate 75 is recirculated through the dialyzer 8 and sorbent filter 36, and thereafter the dialysate is sent back to the same reservoir 17 through the dialysate flow path 54. This recirculation continues as determined by the processor 77 including, but not limited to, because the sorbent filter 36 has been spent, or the dialysate fluid is contaminated, or ultrafiltration has resulted in the reservoir 17 becoming full and requiring that it be drained and refilled. Meanwhile, in the event the fluid in reservoir 20 is contaminated, it is drained through the drain flow path 55, and then the reservoir 20 is refilled using the fresh dialysate flow path 56.

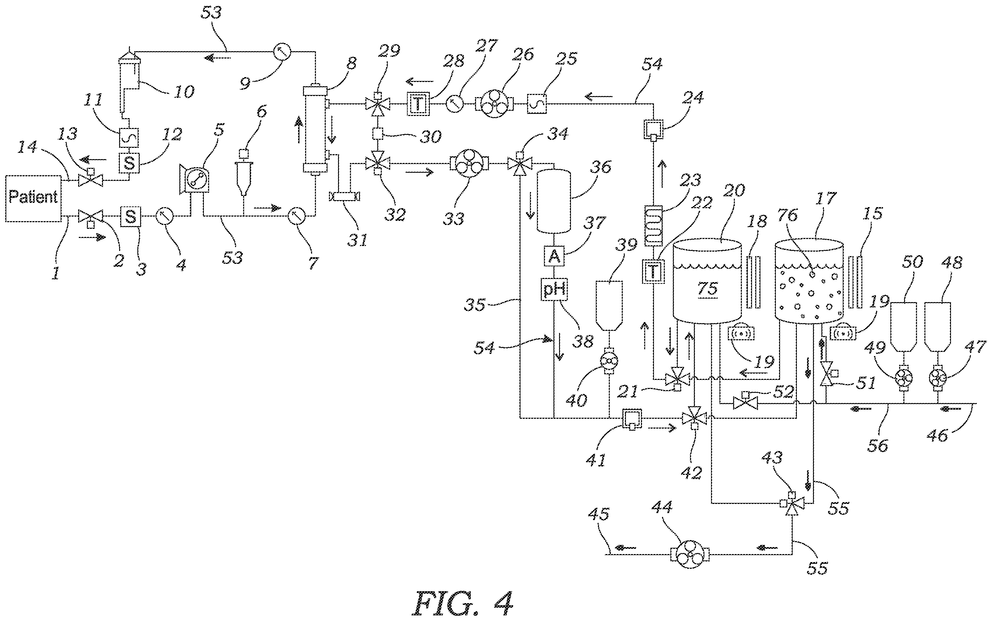

As illustrated in FIG. 4, once the processor 77 has determined that continued use of reservoir 17 for dialysis treatment is not appropriate, the processor 77 switches the various valve assemblies (21, 42, 43, 51 and 52) to remove reservoir 17 from the dialysate flow path 54, and to instead insert reservoir 20 within the dialysis flow path 54 for dialysis treatment. Fresh dialysate 75 is recirculated through the dialyzer 8 and sorbent filter 36 back to the same reservoir 20. Again, this recirculation continues using reservoir 20, as determined by the processor 77, until switching back to reservoir 17, or until dialysis treatment has been completed. While dialysis treatment continues using reservoir 20, contaminated fluid 76 in reservoir 17 is drained through the drain flow path 55. Thereafter, reservoir 17 is refilled using the fresh dialysate flow path 56. Like other treatment methods, this switching back and forth between reservoirs (17 and 20) continues until the dialysis treatment is complete.

In still an additional embodiment, and as illustrated in FIGS. 5 and 6, hemodialysis treatment is conducted in similar manner as illustrated in FIG. 2 in which the sorbent filter 36 is not utilized within the dialysate flow path 54. Though it is possible to utilize the sorbent filter 36 within the dialysate flow path 54, for this embodiment it is preferred that the fresh dialysate 75 be directed through the sorbent filter bypass path 35 so as to avoid the sorbent filter 36. During treatment, the fresh dialysate 75 from the first reservoir (17 or 20) is recirculated past the dialyzer 8 through sorbent filter bypass path 35 and directed back to the same reservoir (17 or 20). Even more preferably for this embodiment, the hemodialysis system does not include sorbent filter 36. Instead, with reference to FIGS. 5 and 6, the hemodialysis system includes a single sorbent filter 71 which is within a separate closed loop flow path referred to herein as the filter flow path 57. Though FIGS. 5 and 6 illustrate the hemodialysis system including two sorbent filters 36 and 71, the sorbent filter 36 within the dialysate flow path 54 is optional and does not need to be incorporated within this embodiment of the hemodialysis system.

Like the prior embodiments, dialysis treatment is implemented while switching back and forth between reservoirs (17 and 20). With reference to FIG. 5, while dialysis treatment uses the fresh dialysate 75 in reservoir 17, the various valve assemblies (21, 42, 43, 51 and 52) are switched to insert the second reservoir 20 into the closed loop filter flow path 57. The contaminated water 76 is drained from the reservoir 20 through pump 58 and pressure sensor 59. Thereafter the contaminated water 76 is filtered through the sorbent filter 71. Reagents 61 and 65 may be introduced into the filter flow path 57 using a gravity feed or pumps 62 and 66. The reagents 61 and 65 are mixed within the mixers 63 and 67 before the now cleaned dialysate is tested for compliance by conductivity testers 64 and 68, ammonium sensor 69, and pH sensor 70. If testing shows the water is now clean, it is directed back to reservoir 20.

With reference to FIG. 6, the processor 77 continues to monitor the output of the various sensors (3, 4, 7, 9, 11, 12, 15, 16, 18, 19, 22, 24, 25, 27, 28, 31, 37, and 59) including those within the dialysate flow path 54. Once the water within reservoir 17 has become contaminated, it is removed from the dialysate flow path 54 and reservoir 20 is substituted in its place by once again switching all of the pertinent valve assemblies (21, 42, 43, 51 and 52). The fresh dialysate 75 from the second reservoir 20 is recirculated in the closed loop dialysate flow path 54 past the dialyzer 8 and directed back to the same reservoir 20. Meanwhile, the now contaminated water 76 in reservoir 17 is drained through pump 58 and pressure sensor 59 before being filtered through the sorbent filter 71. Again, reagents (61 and 65) may be introduced into the filter flow path 57 where the reagents (61 and 65) are mixed within the mixers (63 and 67). The now clean dialysate is tested for compliance by conductivity testers (64 and 68), ammonium sensor 69 and pH sensor 70 before filling reservoir 17. This process of alternating reservoirs (17 and 20) continues until the prescribed hemodialysis treatment is completed, or a fault is detected which requires that treatment be halted.

FIG. 7A illustrates still an additional embodiment of the hemodialysis system which operates in recirculating mode where the dialysate flows in a closed-loop system through the sorbent filter 36. Like other embodiments, the blood flow path 53 transports blood in a closed loop system by connecting to the arterial blood line 1 and venous blood line 14 to a patient for transporting blood from a patient to the dialyzer 8 and back to the patient. Dialysate is stored in a reservoir 17 with the level of dialysate's measured by a fluid mass sensor 19, such as a mass strain gauge or load cell 19, and the dialysate's temperature maintained by a heater 23. Dialysate is recirculated through the dialyzer 8 and sorbent filter 36 using pumps 26 and 33. Thereafter, the dialysate is sent back to the same reservoir 17 through the dialysate flow path 54.

In the embodiment illustrated in FIG. 7A, chemical concentrates sources (48 and 50) are provided which can be added to the clean water, as necessary, to maintain proper chemicals in the dialysate. Preferably, the first reagent source 48 contains salts and the second reagent source 50 contains bicarbonate and lactate solution. The chemical concentrates are introduced into the dialysate flow path 54 using the chemical concentrate pumps (47 and 49) where the clean water and chemical concentrates are mixed with mixers (63 and 67). Again, the dialysate flow path 54 may include a flow sensor 25, one or more pressure sensors 27, and a sample port 79.

In some embodiments, the dialysate flow path 54 also includes a conductivity sensor 41 positioned between the second mixer 67 and reservoir 17, and includes an ammonia sensor 37, a pH sensor 38 and a combined conductivity/temperature sensor 24 positioned between the reservoir 17 and dialyzer 8. A control processor 77 is connected to the various sensors (e.g., 3, 4, 7, 11, 12, 15, 16, 19, 24, 25, and 27) and pumps (5, 6, 26, 33, 44, 47 and 49) to control the hemodialysis treatment.

The embodiment of the hemodialysis system illustrated in FIG. 7A operates in a closed loop recirculating mode where the dialysate flows through the sorbent filter 36. Dialysate is stored in a reservoir 17 and recirculated through the dialyzer 8 and sorbent filter 36. Chemical concentrates are added to the filtered water, as necessary. Recirculation continues as determined by the processor 77 until treatment has completed, the sorbent filter 36 has been spent, the dialysate fluid is contaminated, or ultrafiltration has resulted in the reservoir 17 becoming full and requiring that it be drained.

Reagent sources (48 and 50) can contain the same or different infusate/reagent solutions having one or more of the following chemical compounds: calcium acetate, calcium chloride, magnesium acetate, magnesium chloride, potassium acetate, potassium chloride, sodium bicarbonate, and sodium carbonate. One or more of these compounds are infused with the dialysate coming out of the sorbent filter 36 to replenish essential sodium ions in the dialysate while also balancing the pH of the dialysate. In this way, the pH of the dialysate can be controlled to closely match with the pH of blood. For example, if the pH of the dialysate falls under 6.5, the reagent solution from one or more of the reagent sources (48 and 50) can be added to the dialysate flow path 54 after the sorbent filter 36 to bring the pH back to the desired level. This process works because fluid leaving the sorbent filter 36 at lower pH generally needs more sodium reinfused than fluid at a higher pH.

According to the invention as claimed, a first reagent source contains a first reagent solution which is a sodium carbonate solution.

In some embodiments, the reagent solution in one of the reagent sources (48 or 50) can have the following compounds: calcium chloride (CaCl₂), magnesium chloride (MgCl₂), and potassium acetate (KAc). The reagent solution can have the following compound concentrations (approximately): CaCl₂ 25 - 40 mM millimolar); MgCl₂ 12.5 - 20 mM; and KAc 75 - 120 mM. In an exemplar embodiment, the reagent solution have the following compound concentrations (approximately): CaCl₂ - 32.04 mM (millimolar); MgCl₂ - 16.02 mM; and KAc - 96.12 mM. It should be noted that other molarities can also be used as long as the approximate molar ratio of each compound is maintained.

In some embodiments, the reagent solution in the other reagent source (reagent source 48 or 50) can be a solution of sodium carbonate (Na₂CO₃). The concentration of the sodium carbonate solution can be approximately 1.5 M. Indeed, sodium carbonate is considered one of the most essential salts due to its highly basicity. Specifically, sodium carbonate includes two molecules of sodium per compound. In this way, sodium can be replenished into a system as necessary, while balancing out the system's pH when the system falls below a desired value, e.g., pH of 7.0. Thus, sodium carbonate is the preferred reagent because each mole of Na₂CO₃ can turn one mole of CO₂ into sodium bicarbonate (NaHCO₃) which is closer to a safe and physiologic pH range in the dialysate.

Specifically, in some preferred embodiments, reagent source 48 can be the solution of CaCl₂, MgCl₂, and KAc, and the reagent source 50 can be the reagent solution of Na₂CO₃. In this embodiment, reagent source 48 can be 3-4 L and reagent source 50 can be 0.5-1.0 L. However, other volumes are possible as long as the ratio is maintained. Alternatively, reagent source 48 can be the solution of Na₂CO₃, and the reagent source 50 can the reagent solution of CaCl₂, MgCl₂, and KAc. In some embodiments, reagent sources (48 and 50) can be combined into a single reagent source having an reagent solution with one or more of the following chemical compounds: calcium acetate, calcium chloride, magnesium acetate, magnesium chloride, potassium acetate, potassium chloride, sodium bicarbonate, and sodium carbonate.

As shown in FIG. 7A, reagent solutions from reagent source 48 and reagent source 50 are added to the dialysate flow path 54 after the sorbent filter 36. The reagent solutions from reagent sources (48 and 50) can enter the dialysate flow path 54 at the same location or at different locations and are mixed with one or more mixers (63 or 67).

In some embodiments, the reagent solution from reagent source 48 is inserted into the dialysate flow path 54 before the first mixer 63, and the reagent solution from reagent source 50 is inserted into the dialysate flow path 54 after the first mixer 63. Once the second reagent solution is inserted into the dialysate flow path 54, the dialysate and reagent solution in the dialysate flow path 54 are mixed again using a second downstream mixer 67 (e.g., second mixer 67).

In the embodiment where the reagent solutions from reagent sources (48 and 50) enter the dialysate flow path 54 at the same location, a single mixer can be used after the injection point. Alternatively, two or more mixers can be used at various locations downstream of the sorbent filter 36 but before dialysate reservoir 17. It should be noted that the dialysate flow path 54 can have a second reservoir to store new and/or refreshed dialysate-dialysate with renewed essential minerals content.

FIG. 8 illustrates a feedback system 800 for monitoring and controlling the concentration of essential minerals (e.g., sodium) in the dialysate after the reagent solution is introduced and mixed in accordance with some embodiments. System 800 includes one or more reagent solution-injection locations 805, mixer 63, a conductivity sensor 41, and an electrode 1010. In some embodiments, and as shown in FIG. 8, the solution-injection locations 805 can be upstream of the dialysate quality sensor 700. The reagent solution from the one or more reagent sources (48 and 50) (not shown in FIG. 8) can be injected into the dialysate flow path 54 using one or more pumps 810. Once the reagent solution is injected, the mixer(s) 63 can be used to mix the reagent solution with the dialysate to achieve homogeneity, hereinafter can be referred to as the "mixed-solution." The conductivity sensor 41 is then used to measure the conductivity value of the mixed-solution, which is then used to determine the level of sodium or sodium ions in the mixed-solution. The conductivity sensor 41 can be pre-calibrated such that a certain conductivity value is expected given an optimum level of sodium ions in the mixed-solution. In some embodiments, the optimum sodium concentration can be between 130 and 145 mM. Specifically, in some exemplar embodiments, the optimum sodium concentration is 140 mM. In some exemplar embodiments, the reagent infused by pump 810 must contain sodium, as depicted in FIG. 8. In the optimal configuration it contains sodium carbonate. For example, if the conductivity value of the mixed-solution is less than the expected conductivity value for the optimum level of sodium ions in the mixed-solution, then a feedback signal can be send to the controller (not shown) that controls the one or more pump 810 to increase the reagent solution injection rate. Alternatively, if the measured conductivity value is higher than the optimum conductivity value, then the reagent solution injection rate can be reduced.

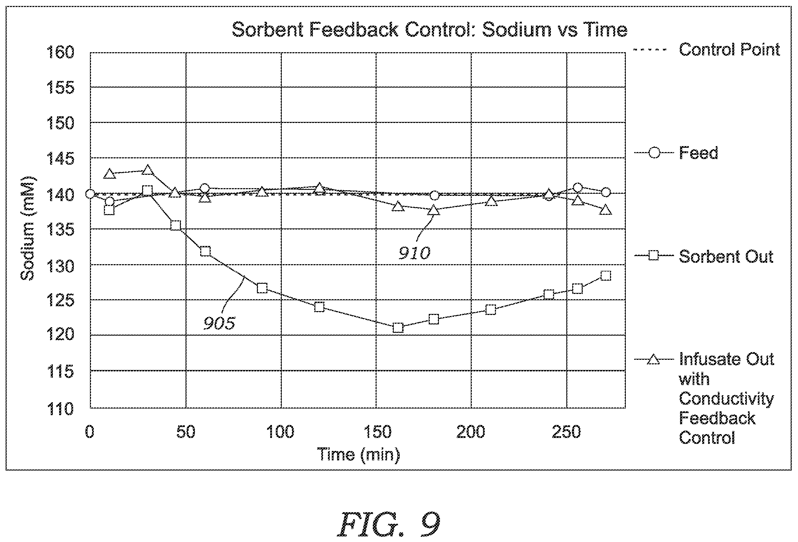

FIG. 9 is a chart illustrating measured sodium concentration using the previously described feedback system 800. Line 905 represents the measured sodium content of the dialysate immediately after the sorbent filter 36. As shown, if left unreplenished, the sodium content of the dialysate at the output of the sorbent filter 36 can dramatically fall as the treatment time progresses. Line 910 represents the measured sodium content of the replenished mixed-solution (after the reagent solution is injected and mixed) using feedback from the conductivity sensor 41. As shown, the sodium replenishing system is able to keep the sodium concentration around the optimum value of 140 mM.

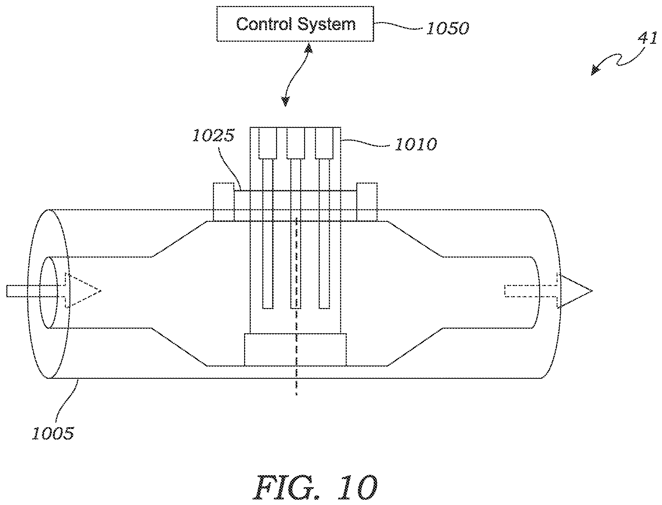

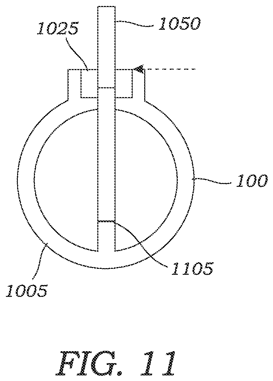

FIG. 10 illustrates the conductivity sensor 41 in accordance with some embodiments of the present disclosure. FIG. 11 illustrates a cross-sectional view of the conductivity sensor 41 at section A. Both FIGS. 10 and 11 will be discussed concurrently. The conductivity sensor 41 includes a sensor body 1005, and one or more electrodes 1010 and a control system 1050. The electrode(s) 1005 can be disposed into a slot 1105 of the sensor body 1005. The electrode(s) 1010 can be secured into the slot 1105, and the slot 1105 sealed using adhesive 1025.

The electrode(s) 1010 can be disposed in the center of sensor body 1005, as best illustrated in FIG. 10. The electrode(s) 1010 can be coupled to the control system 1050, which is coupled to the one or more pumps (e.g., pump 47, pump 49, and pump 810) (not shown) so that the amount of reagent solution injected into the dialysate flow path 54 can be controlled. In some embodiments, control system 1050 can include processor 77 ( shown in FIG. 8) configured to determine the conductivity value based on readings from the electrode(s). The conductivity value is then used to control the amount of reagent solution being injected into the dialysate flow path 54. This establishes a feedback control loop between the one or more pumps (47, 49, 810) and the conductivity sensor 41. In this way, the one or more pumps (47, 49, 810) can control the rate of injection of the one or more reagent solution from reagent source 48 and/or reagent source 50 to maintain a consistent level of sodium in the mixed-solution.

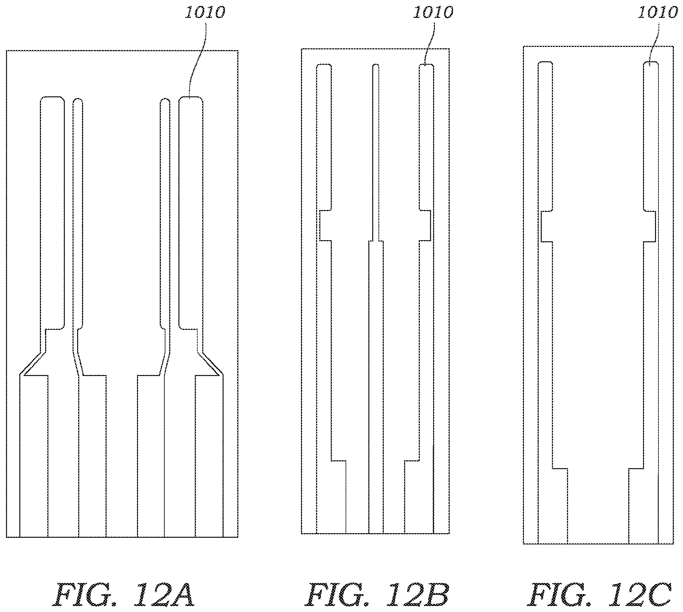

FIGS. 12A-C illustrate exemplar embodiments of various electrode 1010 designs that can be implemented in the conductivity sensor 41. Each electrode(s) 1010 is configured to measure the conductivity of the mixed-solution. The electrode(s) 1010 used for measuring conductivity may be composed of stainless steel, graphite, inconel, titanium, gold, platinum, palladium, or other non-corrosive, electrically conductivte biocompatible material. In some embodiments the electrode(s) 1010 may take the form of rods, plates, disks, or cylinders in the form of a plurality of electrodes across which conductivity can be measured as known to those skilled in the art. In some exemplar embodiments, the electrode(s) 1010 can take the form of rods, plates, disks, or cylinders. Additionally, and as illustrated in FIGS. 12A-12C, respectively, the electrodes can take the form of four, three, or two pole electrodes 1010 across which conductivity can be measured as known to those skilled in the art. Various other electrode forms and configurations can be determined by those skilled in the art.

In some embodiments, the electrode(s) 1010 can be a two dimensional and adhered onto an electrically insulated backing by an additive process. Specifically, the electrode(s) 1010 can printed with conductive materials or inks onto a planar surface with screen printing or sputter coating, or other similar processes of creating two dimensional conductive shapes on a surface. In some embodiments, the two dimensional electrode(s) 1010 can be created by a removal process, such as a laser ablation, chemical etching, or mechanical removal. These two dimensional electrode(s) 1010 can be printed on ceramics including but not limited to zirconium oxide and glass. Further, in some embodiments, the electrode(s) 1010 can be printed on different polymers, including but not limited to acrylic, polycarbonate, or polyester.

In closing, regarding the exemplary embodiments of the present disclosure as shown and described herein, it will be appreciated that a hemodialysis system is disclosed. The principles of the present disclosure may be practiced in a number of configurations beyond those shown and described, so it is to be understood that the invention is not in any way limited by the exemplary embodiments, but is generally directed to a hemodialysis system and is able to take numerous forms to do so without departing from the scope of the present disclosure. It will also be appreciated by those skilled in the art that the present disclosure is not limited to the particular geometries and materials of construction disclosed, but may instead entail other functionally comparable structures or materials, now known or later developed, without departing from the scope of the present disclosure. Furthermore, the various features of each of the above-described embodiments may be combined in any logical manner and are intended to be included within the scope of the present disclosure.

Groupings of alternative embodiments, elements, or steps of the present disclosure are not to be construed as limitations. Each group member may be referred to individually or in any combination with other group members disclosed herein. It is anticipated that one or more members of a group may be included in, or deleted from, a group for reasons of convenience. When any such inclusion or deletion occurs, the specification is deemed to contain the group as modified.

Unless otherwise indicated, all numbers expressing a characteristic, item, quantity, parameter, property, term, and so forth used in the present specification and claims are to be understood as being modified in all instances by the term "about." As used herein, the term "about" means that the characteristic, item, quantity, parameter, property, or term so qualified encompasses a range of plus or minus ten percent above and below the value of the stated characteristic, item, quantity, parameter, property, or term. Accordingly, unless indicated to the contrary, the numerical parameters set forth in the Specification and attached claims are approximations that may vary. At the very least, and not as an attempt to limit the scope of the claims, each numerical indication should at least be construed in light of the number of reported significant digits and by applying ordinary rounding techniques. Notwithstanding that the numerical ranges and values setting forth the broad scope of the present disclosure are approximations, the numerical ranges and values set forth in the specific examples are reported as precisely as possible. Any numerical range or value, however, inherently contains certain errors necessarily resulting from the standard deviation found in their respective testing measurements. Recitation of numerical ranges of values herein is merely intended to serve as a shorthand method of referring individually to each separate numerical value falling within the range. Unless otherwise indicated herein, each individual value of a numerical range is incorporated into the present Specification as if it were individually recited herein.

The terms "a," "an," "the" and similar referents used in the context of describing the present disclosure (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the present invention and does not pose a limitation on the scope of the invention otherwise claimed. No language in the present specification should be construed as indicating any non-claimed element essential to the practice of the invention.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of the present invention so claimed are inherently or expressly described and enabled herein.

It should be understood that the logic code, programs, modules, processes, methods, and the order in which the respective elements of each method are performed are purely exemplary. Depending on the implementation, they may be performed in any order or in parallel, unless indicated otherwise in the present disclosure. Further, the logic code is not related, or limited to any particular programming language, and may comprise one or more modules that execute on one or more processors in a distributed, non-distributed, or multiprocessing environment.

While several particular forms of the invention have been illustrated and described, it will be apparent that various modifications can be made without departing from the scope of the invention, which is defined by the appended claims. Therefore, it is not intended that the invention be limited except by the following claims.

## Claims

1. A system for replenishing minerals of dialysate in a dialyzer, the system comprising:
a sorbent filter (71) configured to remove ammonia from the dialysate, the sorbent filter having an outlet that outputs the dialysate to a dialysate flow path (54);
a first reagent source (50) containing a first reagent solution which is a sodium carbonate solution
a first pump (49) configured to inject the first reagent solution into the dialysate flow path of the sorbent filter;
a first mixer (67) coupled to the dialysate flow path and downstream of the first pump, the first mixer configured to mix the dialysate with the first reagent solution;
a conductivity sensor (41) configured to measure the level of dissolved solids in the dialysate after the first mixer; and
a controller (77) configured to adjust a flow rate of the first reagent solution by adjusting the first pump based at least on the level of dissolved solids in the dialysate.

2. The system of claim 1, wherein the conductivity sensor comprises a sodium level sensor configured to measure the level of sodium in the dialysate.

3. The system of claim 2, wherein the controller is further configured adjust a flow rate of the first reagent solution by adjusting the first pump based at least on the level of sodium in the dialysate.

4. The system of claim 1, wherein the sodium carbonate solution has a concentration of approximately 1.5 M.

5. The system of claim 1, further comprising:
a second reagent source (48) containing a second reagent solution comprising a solution of a plurality of mineral compounds; and
a second pump (47) configured to inject the second reagent solution into the dialysate flow path of the sorbent filter, wherein the second pump is located upstream of the first pump.

6. The system of claim 5, further comprising a second mixer (63) disposed upstream of the first mixer, wherein the second mixer is configured to mix the dialysate with the second reagent solution before first reagent is injected into the dialysate flow path by the first pump.

7. The system of claim 5, wherein the second reagent solution comprises a solution of calcium chloride (CaCl₂), magnesium chloride (MgCl₂), and potassium acetate (KAc).

8. The system of claim 7, wherein the CaCl₂ in the second reagent solution has a concentration between 25 and 40 millimolar (mM), wherein the MgCl₂ in the second reagent solution has a concentration between 12.5 and 20 mM, and wherein the KAc in the second reagent solution has a concentration between 75 and 120 mM.

9. The system of claim 7, wherein the CaCl₂ in the second reagent solution has a concentration of 32.04 mM, wherein the MgCl₂ in the second reagent solution has a concentration of 16.02 mM, and wherein the KAc in the second reagent solution has a concentration of 96.12 mM.

10. The system of claim 5, wherein the first reagent source is between 1 and 3 liters, and the second reagent source is between 0.5 and 1.0 liters.

11. The system of claim 5 wherein the controller is further configured to adjust a flow rate of the second reagent solution by adjusting the second pump based at least on the level of sodium in the dialysate.

12. The system of claim 1, wherein the conductivity sensor is further configured to measure a conductivity value of the dialysate, optionally wherein the conductivity sensor is pre-calibrated such that a certain conductivity value is expected given an optimum level of sodium in the dialysate, optionally wherein the optimum level of sodium concentration is between 130 and 145 mM.

13. The system of claim 5, wherein the conductivity sensor comprises a sensor body, an electrode, and control system, optionally wherein the electrode is coupled to the control system, wherein the control system is coupled to the first pump and the second pump, and wherein the control system is configured to control the amount of first reagent solution and second reagent solution into the dialysate flow path, optionally wherein the control system comprises a processor, wherein the processor is configured to determine a conductivity value of the dialysate based on readings from the electrode, and wherein the determined conductivity value is configured to control the amount of first reagent solution and second reagent solution into the dialysate flow path so as to establish a feedback control loop (800) between the first pump, the second pump, and the conductivity sensor.

14. The system of claim 13, wherein the electrode comprises a two, three, or four pole electrode configured to measure a conductivity value of the dialysate, or wherein the electrode is disposed into a slot of the sensor body, and wherein the slot is sealed using adhesive and is configured to secure the electrode housed within the sensor body.

## Patentansprüche

1. System zum Wiederauffüllen von Mineralien von Dialysat in einem Dialysator, wobei das System umfasst:
einen Sorbensfilter (71), der dazu konfiguriert ist, Ammoniak aus dem Dialysat zu entfernen, wobei der Sorbensfilter einen Auslass aufweist, der das Dialysat an einen Dialysatflussweg (54) ausgibt;
eine erste Reagensquelle (50), enthaltend eine erste Reagenslösung, die eine Natriumcarbonatlösung ist
eine erste Pumpe (49), die dazu konfiguriert ist, die erste Reagenslösung in den Dialysatflussweg des Sorbensfilters einzuspritzen;
einen ersten Mischer (67), der an den Dialysatflussweg gekoppelt und stromabwärts von der ersten Pumpe ist, wobei der erste Mischer dazu konfiguriert ist, das Dialysat mit der ersten Reagenslösung zu mischen;
einen Leitfähigkeitssensor (41), der dazu konfiguriert ist, den Pegel von gelösten Feststoffen in dem Dialysat nach dem ersten Mischer zu messen; und
eine Steuereinheit (77), die dazu konfiguriert ist, eine Flussrate der ersten Reagenslösung durch Justieren der ersten Pumpe auf der Basis von mindestens dem Pegel von gelösten Feststoffen in dem Dialysat zu justieren.

2. System nach Anspruch 1, wobei der Leitfähigkeitssensor einen Natriumpegelsensor umfasst, der dazu konfiguriert ist, den Pegel von Natrium in dem Dialysat zu messen.

3. System nach Anspruch 2, wobei die Steuereinheit ferner dazu konfiguriert ist, eine Flussrate der ersten Reagenslösung durch Justieren der ersten Pumpe auf der Basis von mindestens dem Pegel von Natrium in dem Dialysat zu justieren.

4. System nach Anspruch 1, wobei die Natriumcarbonatlösung eine Konzentration von ungefähr 1,5 M aufweist.

5. System nach Anspruch 1, ferner umfassend:
eine zweite Reagensquelle (48), enthaltend eine zweite Reagenslösung, umfassend eine Lösung einer Vielzahl von Mineralverbindungen; und
eine zweite Pumpe (47), die dazu konfiguriert ist, die zweite Reagenslösung in den Dialysatflussweg des Sorbensfilters einzuspritzen, wobei sich die zweite Pumpe stromaufwärts von der ersten Pumpe befindet.

6. System nach Anspruch 5, ferner umfassend einen zweiten Mischer (63), der stromaufwärts von dem ersten Mischer angeordnet ist, wobei der zweite Mischer dazu konfiguriert ist, das Dialysat mit der zweiten Reagenslösung zu mischen, bevor erstes Reagens durch die erste Pumpe in den Dialysatflussweg eingespritzt wird.

7. System nach Anspruch 5, wobei die zweite Reagenslösung eine Lösung von Calciumchlorid (CaCl₂), Magnesiumchlorid (MgCl₂) und Kaliumacetat (KAc) umfasst.

8. System nach Anspruch 7, wobei das CaCl₂ in der zweiten Reagenslösung eine Konzentration zwischen 25 und 40 Millimol (mM) aufweist, wobei das MgCl₂ in der zweiten Reagenslösung eine Konzentration zwischen 12,5 und 20 mM aufweist, und wobei das KAc in der zweiten Reagenslösung eine Konzentration zwischen 75 und 120 mM aufweist.

9. System nach Anspruch 7, wobei das CaCl₂ in der zweiten Reagenslösung eine Konzentration von 32,04 mM aufweist, wobei das MgCl₂ in der zweiten Reagenslösung eine Konzentration von 16,02 mM aufweist, und wobei das KAc in der zweiten Reagenslösung eine Konzentration von 96,12 mM aufweist.

10. System nach Anspruch 5, wobei die erste Reagensquelle zwischen 1 und 3 Liter beträgt und die zweite Reagensquelle zwischen 0,5 und 1,0 Liter beträgt.

11. System nach Anspruch 5, wobei die Steuereinheit ferner dazu konfiguriert ist, eine Flussrate der zweiten Reagenslösung durch Justieren der zweiten Pumpe auf der Basis von mindestens dem Pegel von Natrium in dem Dialysat zu justieren.

12. System nach Anspruch 1, wobei der Leitfähigkeitssensor ferner dazu konfiguriert ist, einen Leitfähigkeitswert des Dialysats zu messen, optional wobei der Leitfähigkeitssensor vorkalibriert ist, so dass ein bestimmter Leitfähigkeitswert angesichts eines optimalen Pegels von Natrium in dem Dialysat erwartet wird, optional wobei der optimale Pegel einer Natriumkonzentration zwischen 130 und 145 mM liegt.

13. System nach Anspruch 5, wobei der Leitfähigkeitssensor einen Sensorkörper, eine Elektrode und ein Steuersystem umfasst, optional wobei die Elektrode an das Steuersystem gekoppelt ist, wobei das Steuersystem an die erste Pumpe und die zweite Pumpe gekoppelt ist, und wobei das Steuersystem dazu konfiguriert ist, die Menge von erster Reagenslösung und zweiter Reagenslösung in den Dialysatflussweg zu steuern, optional wobei das Steuersystem einen Prozessor umfasst, wobei der Prozessor dazu konfiguriert ist, einen Leitfähigkeitswert des Dialysats auf der Basis von Messwerten von der Elektrode zu bestimmen, und wobei der bestimmte Leitfähigkeitswert dazu konfiguriert ist, die Menge von erster Reagenslösung und zweiter Reagenslösung in den Dialysatflussweg zu steuern, um eine Rückmeldungssteuerschleife (800) zwischen der ersten Pumpe, der zweiten Pumpe und dem Leitfähigkeitssensor einzurichten.

14. System nach Anspruch 13, wobei die Elektrode eine Zwei-, Drei- oder Vier-Pol-Elektrode umfasst, die dazu konfiguriert ist, einen Leitfähigkeitswert des Dialysats zu messen, oder wobei die Elektrode in einem Schlitz des Sensorkörpers angeordnet ist, und wobei der Schlitz unter Verwendung von Klebstoff versiegelt wird und dazu konfiguriert ist, die Elektrode zu sichern, die innerhalb des Sensorkörpers untergebracht ist.

## Revendications

1. Système de réalimentation en minéraux de dialysat dans un dialyseur, le système comprenant :
un filtre à sorbant (71) configuré pour éliminer l'ammoniac du dialysat, le filtre à sorbant ayant une sortie qui fait sortir le dialysat au niveau d'un trajet d'écoulement de dialysat (54) ;
une première source de réactif (50) contenant une première solution de réactif qui est une solution de carbonate de sodium ;
une première pompe (49) configurée pour injecter la première solution de réactif dans le trajet d'écoulement de dialysat du filtre à sorbant ;
un premier mélangeur (67) couplé au trajet d'écoulement de dialysat et en aval de la première pompe, le premier mélangeur étant configuré pour mélanger le dialysat avec la première solution de réactif ;
un capteur de conductivité (41) configuré pour mesurer le niveau de solides dissous dans le dialysat après le premier mélangeur ; et
un dispositif de commande (77) configuré pour ajuster un débit de la première solution de réactif en réglant la première pompe au moins sur la base du niveau de solides dissous dans le dialysat.

2. Système selon la revendication 1, dans lequel le capteur de conductivité comprend un capteur de niveau de sodium configuré pour mesurer le niveau de sodium dans le dialysat.

3. Système selon la revendication 2, dans lequel le dispositif de commande est en outre configuré pour ajuster un débit de la première solution de réactif en réglant la première pompe au moins sur la base du niveau de sodium dans le dialysat.

4. Système selon la revendication 1, dans lequel la solution de carbonate de sodium a une concentration d'environ 1,5 M.

5. Système selon la revendication 1, comprenant en outre :
une deuxième source de réactif (48) contenant une deuxième solution de réactif comprenant une solution d'une pluralité de composés minéraux ; et
une deuxième pompe (47) configurée pour injecter la deuxième solution de réactif dans le trajet d'écoulement de dialysat du filtre à sorbant, la deuxième pompe étant située en amont de la première pompe.

6. Système selon la revendication 5, comprenant en outre un deuxième mélangeur (63) disposé en amont du premier mélangeur, dans lequel le deuxième mélangeur est configuré pour mélanger le dialysat avec la deuxième solution de réactif avant l'injection de premier réactif dans le trajet d'écoulement de dialysat par la première pompe.

7. Système selon la revendication 5, dans lequel la deuxième solution de réactif comprend une solution de chlorure de calcium (CaCl₂), de chlorure de magnésium (MgCl₂) et d'acétate de potassium (KAc).

8. Système selon la revendication 7, dans lequel le CaCl₂ dans la deuxième solution de réactif a une concentration comprise entre 25 et 40 millimolaires (mM), dans lequel le MgCl₂ dans la deuxième solution de réactif a une concentration comprise entre 12,5 et 20 mM, et dans lequel le KAc dans la deuxième solution de réactif a une concentration comprise entre 75 et 120 mM.

9. Système selon la revendication 7, dans lequel le CaCl₂ dans la deuxième solution de réactif a une concentration de 32,04 mM, dans lequel le MgCl₂ dans la deuxième solution de réactif a une concentration de 16,02 mM, et dans lequel le KAc dans la deuxième solution de réactif a une concentration de 96,12 mM.

10. Système selon la revendication 5, dans lequel la première source de réactif est comprise entre 1 et 3 litres, et la deuxième source de réactif est comprise entre 0,5 et 1,0 litre.

11. Système selon la revendication 5, dans lequel le dispositif de commande est en outre configuré pour ajuster un débit de la deuxième solution de réactif en réglant la deuxième pompe au moins sur la base du niveau de sodium dans le dialysat.

12. Système selon la revendication 1, dans lequel le capteur de conductivité est en outre configuré pour mesurer une valeur de conductivité du dialysat, éventuellement dans lequel le capteur de conductivité est préétalonné de sorte qu'une certaine valeur de conductivité est attendue étant donné un niveau optimal de sodium dans le dialysat, éventuellement dans lequel le niveau optimal de concentration en sodium est compris entre 130 et 145 mM.

13. Système selon la revendication 5, dans lequel le capteur de conductivité comprend un corps de capteur, une électrode et un système de commande, éventuellement dans lequel l'électrode est couplée au système de commande, dans lequel le système de commande est couplé à la première pompe et à la deuxième pompe, et dans lequel le système de commande est configuré pour contrôler la quantité de première solution de réactif et de deuxième solution de réactif dans le trajet d'écoulement de dialysat, éventuellement dans lequel le système de commande comprend un processeur, dans lequel le processeur est configuré pour déterminer une valeur de conductivité du dialysat sur la base de lectures provenant de l'électrode, et dans lequel la valeur de conductivité déterminée est configurée pour contrôler la quantité de première solution de réactif et de deuxième solution de réactif dans le trajet d'écoulement de dialysat de façon à établir une boucle de commande de rétroaction (800) entre la première pompe, la deuxième pompe et le capteur de conductivité.

14. Système selon la revendication 13, dans lequel l'électrode comprend une électrode à deux, trois ou quatre pôles configurée pour mesurer une valeur de conductivité du dialysat, ou dans lequel l'électrode est disposée dans une fente du corps de capteur, et dans lequel la fente est obturée à l'aide d'un adhésif et est configurée pour fixer l'électrode logée à l'intérieur du corps de capteur.
